# EUROPEAN PATENT SPECIFICATION

(11) **EP 3 324 923 B1**
(45) Date of publication and mention of the grant of the patent: **29.04.2020**
(21) Application number: 16744484.3
(22) Date of filing: 22.07.2016
(51) Int. Cl.: A61K 8/44, A61Q 17/04

(54) **COMPOSITIONS AND METHODS USING PALYTHINE**
ZUSAMMENSETZUNGEN UND VERFAHREN MIT PALYTHIN
COMPOSITIONS ET PROCÉDÉS UTILISANT DE LA PALYTHINE

(30) Priority: 23.07.2015 GB 201512996
(43) Date of publication of application: 30.05.2018
(73) Proprietor: King's College London, London WC2R 2LS (GB)
(72) Inventor: LONG, Paul F., London SE1 9NH (GB); YOUNG, Antony R., London SE1 9RT (GB); LAWRENCE, Karl, London SE1 9RT (GB)
(74) Representative: Script IP Limited
(86) International application number: PCT/GB2016/052227
(87) International publication number: WO 2017/013441

(56) References cited:
- WO-A1-00/24369
- WO-A1-02/39974
- WO-A1-2011/096628
- FR-A1- 2 803 201
- JP-A- S6 064 906
- MAKINO ET AL: "The influence of ultraviolet irradiation on marine organisms. 3. Effects of ultraviolet absorbing substance palythine from Chondrus yendoi", JAPANESE JOURNAL OF PHYCOLOGY (JAPANESE EDITION), JAPANESE SOCIETY OF PHYCOLOGY, JP, vol. 47, no. 3, 10 November 1999 (1999-11-10), pages 173-177, XP009191768, ISSN: 0038-1578 cited in the application

## Description

### FIELD OF THE INVENTION

The invention relates to compositions such as sunscreen compositions comprising palythine, or salts thereof, as the active ingredient.

### BACKGROUND OF THE INVENTION

Sunlight has deleterious effects on human skin, mainly because of the ultraviolet radiation (UVR) part of the electromagnetic spectrum of which sunlight is comprised. There are two regions of UVR which are of most relevance to human health concerns: UVB (from 280-320 nm) and UVA (from 320-400nm). Short-term adverse clinical effects of UVR, caused by acute human exposure, are sunburn (inflammation erythema) and immune suppression. In the long term, accumulative exposure to UVR may result in premature ageing and the development of skin lesions and skin cancer. The incidence of these severe health risks associated with UVR, including all types of skin cancer, continues to increase.

UVR exerts its effects in two ways. First, by direct damage to DNA and other cellular macromolecules, such as lipids and proteins, which absorb wavelengths within the UV range of 295-400 nm. The photomolecular events that result in skin cancer include the generation of DNA photolesions. These lesions produce the characteristic mutations found in skin cancer and, importantly, are readily induced in human epidermis by sub-erythemal doses of UVA and UVB, that is, exposure which is insufficient to cause the appearance of sunburn.

Secondly, UVR also generates reactive oxygen species, unstable oxygen molecules that cause oxidative damage to DNA, which, if not repaired, results in mutations having a role in skin cancer.

UVR causes skin ageing by induction of matrix metalloproteinases in the skin. These enzymes degrade dermal collagen, which is the main structural protein of the skin. The induction of those enzymes has been widely attributed to reactive oxygen species, but there is increasing evidence that UVR-induced DNA damage may also trigger their expression.

It is a problem in the art to protect against UVA, as well as UVB radiation, as well as the oxidative effects of reactive oxygen species.

There is a dearth of new sunscreen products introduced into the market. Each year 75,000 people are diagnosed with, and 10,000 people die from, melanoma in the USA (Sharfstein 2015 NEJM vol 373 pages 101-103). In particular, sunscreens with effective protection against harmful UVA and UVB radiation are needed.

Mycosporine-like amino acids (MAAs) are water-soluble compounds characterized by either a cyclohexenone or cyclohexenimine ring conjugated with the nitrogen substituent of an amino acid. They are found in marine cyanobacteria, plants (algae, seaweeds) and invertebrates, as well as in the skin, ocular lenses and eggs of freshwater and marine teleost fish.

MAAs have been present in known sun filter compositions, although they have been taught as offering protection only in the UVA region of the solar UVR spectrum. Previously known commercial examples of MAA-based skin-care compositions all contain mixtures of MAAs. None of these contain more than trace amounts of palythine in the composition.

An article broadcast on BBC television in early 2014 disclosed that MAAs from corals may be useful as sunscreens. It was mentioned that MAAs can pass up the food chain, for example into fish, or may even be extracted from seaweed. The article did not mention any specific MAA. The article did not disclose any specific formulations.

The molecular effects caused by the sun are accumulative and the use of sun filters is widely advocated to prevent long-term clinical photodamage. Sun filters are well known. Some filters, such as zinc oxide and titanium dioxide act as physical barriers to the sun's rays. Other materials, such as cinnamates, aminobenzoic acid derivatives and benzophenones, absorb UVR. In addition, antioxidants are often also incorporated into skin-care compositions. However, little is known about the long-term toxicity of conventional sun filters. In addition, most commonly used sun filters are not readily biodegradable and may damage fragile marine ecosystems, even at extremely low concentrations.

Bhandari and Sharma (2007 Indian J. Biochem Biophys vol 44 pages 231-9) disclose effect of UV-B and high visual radiation on photosynthesis in freshwater (*Nostoc spongiformae*) and marine (*Phormidium corium*) cyanobacteria.

Helioguard is a known prior art sunscreen composition. Helioguard comprises only 0.003% palythine. Helioguard therefore comprises only trace amounts of palythine.

WO2007/026037 describes a mixture of MAAs (asterin-330 + palythine) which can be used as an antioxidant and which is suitable for use in the biotechnology industry. The potential use of a purified extract of MAAs as an antioxidant substance, specifically asterin-330 + palythine isolated from red alga *Gelidium sesquipedale*, for the prevention of oxidative stress is disclosed. In more detail, WO2007/026037 discloses a purified mixture of micosporine-like amino acids asterina-330 and palythine extracted of the red alga *Gelidium sesquipedale* (Merciful) Thuret et Bornet to be enriched in asterine-330 against palythine (proportion 6:1), not to present/ display activity significant antioxidant like inhibitor of the production of water soluble free radicals in the rank of pH 6 - 8.5, to present/display to a concentration of 10 µM an activity antioxidant concerning inhibition of the lipidic peroxidation next to 50% of which it corresponds to a similar concentration of α-tocopherol, and to present/ display significant activity antioxidant concerning kidnapping of radicals superoxide to equal concentration or 50 superior to µM, inhibiting to 50% the kinetic oxidation of pyrogallol when the concentration of the purified mixture is of 200 µM. In these compositions, palythine is included in only trace amounts i.e. 0.1% or less. In fact, in a 200 µM mixture of 6:1 Asterine 330:palythine, molecular weight of asterine is 288, palythine is 244. So a 200 microM mixture of 6:1 asterine:palythine contains 167 microM asterine and 33 microM palythine. 167 microM asterine contains 0.048 g/L = 0.005 % (g/ 100 mL); 33 microM palythine contains 0.008 g/L = 0.0008 % (g/ 100 mL). Therefore, in these compositions, palythine is included in only 0.0008 %.

WO2013/067543 describes methods and compositions for administering a specific wavelength phototherapy. Palythine is mentioned as one of multiple active ingredients applied to block certain wavelengths yet allow other wavelengths to pass through. The focus of this document is on administering phototherapy. There is no mention of sunscreens, except at paragraph [0007] where it is mentioned that allowing certain UVB radiation to pass is desirable.

WO2011/ 158041 describes a personal care composition comprising a microalgae extract from *Chlorogloeopsis* spp. It is mentioned at paragraph 6 that "However, UV damage may still be caused to human keratinous tissue where the tissue is exposed to said radiation due to insufficient protection provided by the sunscreens or other sun protective materials applied to the affected areas." Therefore this document teaches away from use of palythine in sunscreens.

WO 2000/024369 (also published as CA 2251457) describes sunscreen compositions for humans including naturally occurring sunscreen agents from plants, algae, cyanobacteria, fungi and bacteria that protect against exposure to solar radiation. It is mentioned that an amino acid may be present and it is mentioned in claim 8 that the amino acid may comprise a mycosporine amino acid, and it is mentioned in claim 9 that the amino acid may be palythine. However, it is clear from claim 12 that even when the amino acid is present and even when that amino acid is palythine, it is present at only 1mg (= 0.1%).

Helioguard 365 is described as "a natural and safe UV-screening compound. Ideal for everyday use - to protect skin from photo-aging.". Helioguard 365 is described as being based on mycosporine-like amino acids from the red alga *Porphyra umbilicalis* that are encapsulated into liposomes to increase their uptake by the skin. *Porphyra umbilicalis*, an organism that lives in shallow water or intertidal region where it is exposed to extreme UVR, produces the most powerful UV-absorbing substances in nature. The isolated mycosporine-like amino acids absorb maximally at 335 nm with a molar absorption coefficient of 45'000. Helioguard 365 is supplied by Mibelle AG Biochemistry, Switzerland (Mibelle Biochemistry, Bolimattstrasse 1, CH-5033 Buchs, Switzerland). In these compositions, palythine is included in only trace amounts i.e. 0.1% or less.

dr. brandt previously marketed a lotion called "UV SPF 30 high protection for face". This was described as a light-weight lotion with dry-touch technology formulated to provide advanced broad spectrum protection and leave a soft powdery finish. It was said that the lotion helps prevent photo-aging, protects skin against other environmental hazards, hypoallergic and fragrance free and was water resistant for 80 minutes. In these compositions, palythine is included in only trace amounts i.e. 0.1% or less if used according to Mibelle, who provide the following description:
*"Help decrease pigmentation induced by UV exposure and prevent photo-ageing with Dr. Brandt UV SPF 30 High Protection For Face Tinted, an advanced broad spectrum UV lotion with a hint of tint for a sun-kissed glow that quickly absorbs. Formulated with detoxophane, a Swiss garden cress sprout that helps defend against pollution and other environmental hazards; dry touch technology, the latest form ulation in an oil-free technology with a soft touch powdery finish; helioguard 365, a natural UVA filter for daily protection that protects the skin from damaging UVA rays which cause photo ageing; and sepicalm VG, nymphaea that helps decrease pigmentation induced by UV exposure, preventing photo ageing, Dr. Brandt UV SPF 30 High Protection For Face Tinted is hypoallergenic and fragrance free and water resistant for 80 minutes.*
*Directions of use:*
- *Apply liberally to face, neck and ears.*
- *Use daily after moisturiser and before sun exposure.*
- *Re-apply every 2-3 hours when outdoors.*
- *Avoid contact with eyes.*"

FR2803201 (Gelyma) describes a seaweed extract having a filter activity with respect to ultraviolet radiation. Mycosporine-like amino acids (MAAs) are mentioned to each absorb at a defined wavelength in the UVB region. Soluble extracts of red algae are described which contain MAAs and have properties of skin protective filter for UVR. A composition called 'Helionori' has these MAAs. In these compositions, palythine is included at a maximum of 0.06% i.e. palythine is present in only trace amounts or insignificant amounts i.e. inadequate for blocking of damage. Their data is based on 0.06% palythine, but they recommend using up to 4% of the MAA mixture - even at this level palythine is only present at 0.12%.

Schmid et al 2003 (SOFW-Journal 129. Jahrgang 7-2003 pages 1 to 5 "Mycosporine-like amino acids: Natural UV-screening compounds from red algae to protect the skin against photoaging") disclose study results on the efficacy of a cream with isolated MAAs from *Porphyra umbilicalis* to prevent photoaging induced by UVA irradiation. No mention of palythine is disclosed. In any case, the final concentration of MAAs in the compositions used on humans did not exceed 0.005%.

WO2002/039974 (also published as European patent application 1341514) discloses personal care compositions comprising compounds which are mycosprorine-like amino acids (MAAs). It is stated therein (4^{th} paragraph) that "MAAs are not suitable to be the sole suncreening agent in sunscreen compositions, but do offer benefits when used in conjunction with a conventional sunscreening agent". Although palythine is mentioned as a possible MAA, it is clear that WO2002/039974 does not provide a disclosure of palythine as an effective sun filter, as the properties of palythine are inconsistent with that quoted statement. WO2002/039974 teaches away from the use of palythine in its own right.

FR1250912 (BLC Thalgo Cosmetic Lab, inventors Sirop and Pradines; Filing Date: 2012-01-31; Priority Number and Date: FR12509122012-01-31) disclose Cosmetic composition photoprotectrice, including/understanding an absorbing agent of ultraviolet radiations and an agent antiradicallaire, two agents being extracts of marine algas. In particular it is disclosed that the absorbing agent of ultraviolet radiations is an extract of a marine alga including/understanding of the amino-acids of mycosporines type (MAAs). More in particular it is disclosed that the amino-acids of type mycosporines (MAAs) of the marine alga include/understand the mycosporine-taurine, the mycosporine-glycine, the palythine, the porphyra-334, the shinorine and/or palythene.). FR1250912 page 3 lines 1-20, states that an aqueous extract of the marine algae Polysiphonia lanosa contain 5 MAAs including palythine. This information is also repeated in the claims on page 21, claims 1-6. FR1250912, page 3 lines 31-35, states the MAA mixture be used at up to 10%, preferably at 8%, with two formulations given on page 19 as 5%. This information is also repeated in the claims on page 22, claim 20. FR1250912, page 9 lines 3-5, states only that the concentrations of each MAA are consistent with each extraction, but nowhere in the entire patent does it give the proportions of the 5 MAAs. The source of *Polysiphonia lanosa* used to make the extract is not given in FR1250912 and is not known to the inventors from the scientific literature nor the supporting material of the manufacturer Thalgo Cosmetics. Therefore, FR1250912 does not provide any enabling disclosure of a sunscreen composition comprising palythine.

In addition the inventors are aware of Makino et al 1999 (Japanese Journal of Phycology vol 47 pages 173-177 "The influence of ultraviolet irradiation on marine organisms. 3. Effects of ultraviolet absorbing substance, palythine from Chondrus yendoi"), WO02/39974 (NERC - see above), FR2803201 (Gelyma - see above), Llewellyn et al 2010 (Marine Drugs vol 8 pages 1273-1291 "Distribution and abundance of MAAs in 33 species of microalgae across 13 classes"), and Rezanka et al 2004 (Folia Microbiologica vol 49 pages 339-352 "Natural microbial UV radiation filters - mycosporine-like amino acids"). Moreover the inventors are aware of Yabe and Minami et al 2000 (Proceedings of Hokkaido Tokai University. Science and engineering 13, 47-50 "Studies on the Ultraviolet Absorbing Substances in Marine Organisms. : 6. The effects of the ultraviolet absorbing substance palythine in the development of spores of Palmaria palmata"). In each of these documents, the palythine concentrations are extremely low, always being below 0.3%. In addition, with particular reference to Makino et al 1999 and to Yabe and Minami et al 2000, these documents deal with spore germination and are of no relevance to the invention. It is not possible to extrapolate from these studies to the field of the invention. Moreover, the inventors believe the UV dose in these studies to be so low as to be irrelevant. The skilled person reading these studies would note that they study spores, not DNA or cells. The studies are of seaweed spores in seawater and as such represent no more than an observation of the natural world. There is no discussion of, nor any teaching towards, sunscreens in these studies.

D1 (WO2011/096628) discloses a method for preparing UV screening nontoxic extract from red algae, and nontoxic sunscreen using same. In more detail, D1 describes preparing *Porphyra tenera* or *Chondrus ocellatus*, adding a solvent mixture comprising a C1-C5 alcohol and water in a ratio of 1:9 to 9:1 by volume to the prepared algae, maintaining the temperature to be 30-60 so as to extract an active ingredient, and filtering the solvent mixture containing the active ingredient.

The present invention seeks to overcome problem(s) associated with the prior art.

### SUMMARY OF THE INVENTION

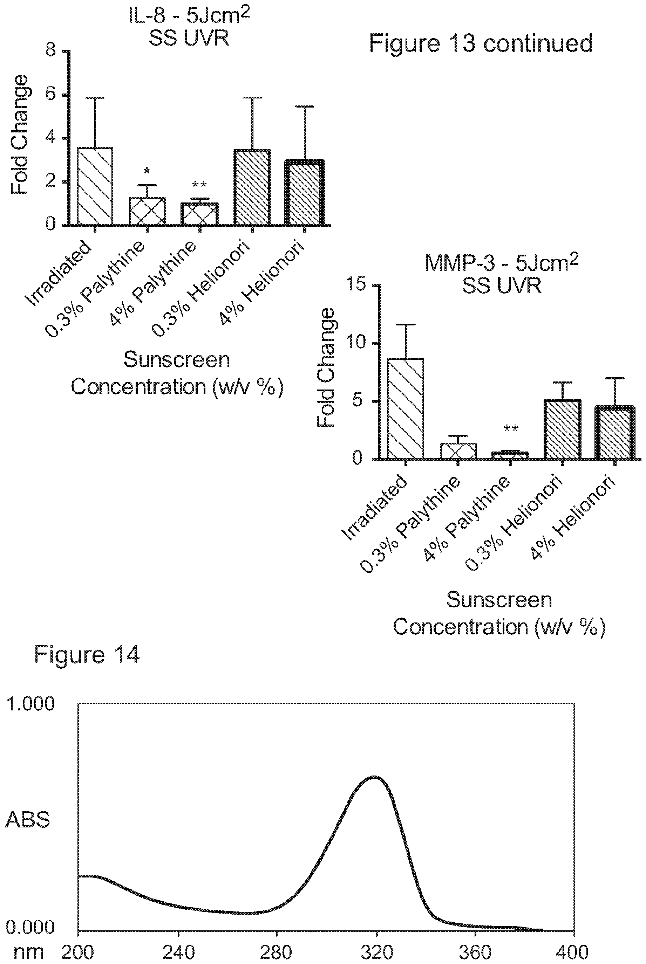

In the prior art, palythine is known to absorb radiation to a very small extent in the UVA spectrum. Figure 14 shows an illustration of palythine absorption. The opinion in the prior art is that this very modest absorption in the UVA spectrum would be inadequate for palythine to be considered a UVAprotectant.

In prior art UVAprotectant compositions such as sunscreens, palythine has been included only at trace amounts insufficient for adequate protection - protection has come from other (non-palythine) active ingredients in prior art compositions.

However, in contrast, the inventors have shown that only a modest level of UVA absorption is required in order to be protective. The inventors teach use of certain concentrations ofpalythine, or salts thereof, as an active ingredient in sunscreen compositions. This is a very surprising finding. The invention is based on these and other remarkable results.

In one aspect, the invention provides a composition comprising:
a sunscreening agent consisting essentially of palythine, or salts thereof, in an amount of 0.3% to 25% by weight.

Thus in one aspect the invention provides a composition comprising palythine in an amount of 0.3% to 25% by weight, wherein palythine is the sole active ingredient.

Suitably said composition is formulated for application to skin.

Suitably said composition is formulated as a moisturiser.

Suitably said composition comprises:
(a) a sunscreening agent consisting essentially of palythine, or salts thereof, in an amount of 0.3% to 25% by weight; and
(b) one or more carriers.

Suitably said composition, optionally, further comprises one or more components selected from:
(c) one or more emulsifiers;
(d) one or more emollients;
(e) water; and
(f) one or more preservatives.

Suitably said composition comprises

| |
|---|
| emulsifier |
| emollient |
| carrier |
| Active (palythine) 0.3 to 25% by weight |
| water |
| preservative |

and optionally further comprises fragrance.
Suitably said composition further comprises antioxidant.
Suitably said composition further comprises gelling agent.
Suitably said composition is a sunscreen composition.
Suitably said composition further comprises liposomal micro-encapsulation. This has the advantage of achieving water resistance. This has the advantage of achieving enhanced skin penetration.
Suitably said composition composition comprises:
a sunscreening agent consisting of palythine, or salts thereof, in an amount of 0.3% to 25% by weight.
In one aspect, the invention relates to a composition as described above for use in the prevention of skin damage, and/ or UVA damage, and/ or skin aging, and/ or UVB damage, and/or reactive oxygen species generation by UVR, wherein said prevention comprises applying said composition to the skin of a subject.
Suitably said administration is topical.

In one aspect, the invention relates to a composition as described above for the prevention of one or more of;
a) UVA damage
b) UVB damage
c) Reactive oxygen species (ROS) damage
d) Formation of cyclobutane pyrimidine dimer (CPD)
e) Formation of 8-oxo-7,8-dihydroguanine (8-oxoGua)
f) Photo-ageing

In one aspect, the invention relates to a composition as described above for the prevention of one or more of;
a) UVA damage
b) UVB damage
c) Reactive oxygen species (ROS) damage
d) Formation of cyclobutane pyrimidine dimer (CPD)
e) Formation of 8-oxo-7,8-dihydroguanine (8-oxoGua)
f) Photo-ageing
g) other UV induced gene expression
h) inflammation
i) oxidative damage

Also described is the use of a composition as described above as a sunscreen, a UVAprotectant, a UVB protectant, or an antioxidant.
In one aspect, the invention relates to a composition as described above for use as a sunscreen, a UVA protectant, a UVB protectant, or an antioxidant.
Also described is use of palythine, or salts thereof, in the manufacture of a composition for prevention of UVA damage, and/ or skin aging, and/ or UVB damage, and/ or reactive oxygen species generation by UVR, wherein said composition comprises palythine, or salts thereof, in an amount of 0.3% to 25% by weight.
Also described is use of palythine in the manufacture of a composition for prevention of UVA damage, and/or skin aging, and/or UVB damage, and/or reactive oxygen species generation by UVR, wherein said composition comprises palythine in an amount of 0.3% to 25% by weight.
Additionally, the invention relates to a composition comprising: a sunscreening agent consisting essentially of palythine, or salts thereof, in an amount of 0.3% to 30% by weight.
Additionally, the invention relates to a composition as described above which comprises

| |
|---|
| emulsifier |
| emollient |
| carrier |
| Active (palythine) 0.3 to 30% by weight |
| water |
| preservative |

and optionally further comprises fragrance.

Also described is use of palythine, or salts thereof, in the manufacture of a composition for prevention of UVA damage, and/ or skin aging, and/ or UVB damage, and/ or reactive oxygen species generation by UVR, wherein said composition comprises palythine, or salts thereof, in an amount of 0.3% to 30% by weight.

### DEFINITIONS

As used herein the term "comprising" means "including at least in part of' and is meant to be inclusive or open ended. When interpreting each statement in this specification that includes the term "comprising", features, elements and/or steps other than that or those prefaced by the term may also be present. Related terms such as "comprise" and "comprises" are to be interpreted in the same manner.
The term "consisting essentially of' limits the scope of a claim to the specified materials or steps "and those that do not materially affect the basic and novel characteristic(s)" of the claimed invention. When the phrase "consisting essentially of' appears in a clause of the body of a claim, rather than immediately following the preamble, it limits only the element set forth in that clause.
The term "consisting of' excludes any element, step, or ingredient not specified in the claim; "consisting of' defined as "closing the claim to the inclusion of materials other than those recited except for impurities ordinarily associated therewith. When the phrase "consists of' appears in a clause of the body of a claim, rather than immediately following the preamble, it limits only the element set forth in that clause; other elements are not excluded from the claim as a whole.
It should be understood that while various embodiments in the specification are presented using "comprising" language, under various circumstances, a related embodiment is also be described using "consisting of' or "consisting essentially of' language.
"Pharmaceutically acceptable" substances refers to those substances which are within the scope of sound medical judgment suitable for use in contact with the tissues of subjects without undue toxicity, irritation, allergic response, and the like, commensurate with a reasonable benefit-to-risk ratio, and effective for their intended use.

### DETAILED DESCRIPTION OF THE INVENTION

The present invention makes use of palythine, or salts thereof. palythine is a member of a class of compounds known as mycosporine-like amino acids (MAAs). It has a CAS Registry Number 67731-19-5, and has the chemical structure shown in Formula 1: 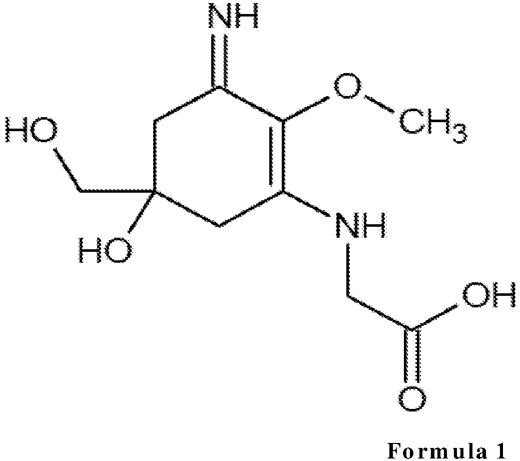 palythine is defined by formula 1 and includes all tautomers, enantiomers. stereoisomers of Formula 1 and mixtures thereof.

Suitably palythine is provided as a free MAA, most suitably as it is extracted directly from seaweed.

Palythine mmr = 244.24; therefore absolute concentrations and % w/v palythine can be interconverted as below:
0.02mM = 0.00002 moles/L palythine
0.00002 x 244.24 = 0.004884g/L / 10 = 0.00048848 g/100ml = 0.00048% w/v palythine
0.04mM = 0.00096% palythine
0.01mM = 0.00024% palythine
0.03mM = 0.00073% palythine
0.05mM = 0.0012% palythine
0.07mM = 0.0017% palythine
0.3% = 12mM palythine
10% = 410mM palythine

The inventors teach that, surprisingly, palythine is very effective as a sun filter and so can be used as the predominant or more suitably the sole active ingredient in compositions such as sun filter or skincare compositions as taught herein. We disclose that palythine is protective against both UVA and UVB radiation and also has antioxidant properties. Thus palythine is unique amongst MAAs as demonstrating UVB and antioxidant protection. Furthermore, palythine is photostable. Those findings make palythine particularly effective as a sun filter. Furthermore, palythine is consumed as a natural constituent of marine
food products and so is known to be non-toxic. Because it is a natural product, palythine, when used in the skin-care compositions of this invention, is not only biodegradable, but also degrades to a non-toxic product, so is fully eco-compatible. The inventors provide new and detailed teachings regarding effective amounts of palythine to incorporate into compositions in order to achieve these beneficial technical effects.

Thus the invention provides the use of palythine as defined by Formula 1 herein, or salts thereof, for the protection of a human or animal against the deleterious effects of ultraviolet radiation.

Prior art compositions have included palythine in trace amounts. Trace amounts means 0.1% or less of the composition. 0.1% palythine is regarded as being 'trace amounts' and insufficient for activity. Suitably amounts of ingredients in compositions are assessed by weight. Thus, suitably trace amounts means 0.1% or less by weight of a composition.

In contrast, the present invention relates to compositions comprising palythine, or salts thereof, in high amounts.
By high amounts it is meant an amount effective to provide a benefit of the invention such as protection from UVB.
By high amounts it is meant an amount effective to provide a benefit of the invention, such as protection from UVA.
By high amounts it is meant an amount effective to provide a benefit of the invention, such as protection from reactive oxygen species generated by UVR.
By high amounts it is meant an amount effective to provide a benefit of the invention, such as anti-ageing protection.
By high amounts it is meant an amount effective to provide a benefit of the invention, such as protection from inflammation.
By high amounts it is meant an amount effective to provide a benefit of the invention, such as protection from DNA damage.

Palythine present in prior art compositions has been present at only insignificant amounts. By insignificant amounts it is meant an amount inadequate for blocking of damage such as UVB and UVA damage and/or reactive oxygen species damage. By contrast, the invention teaches incorporation of palythine, or salts thereof, into compositions in amounts effective in blocking of UVB and UVA and/or reactive oxygen species damage.

Blocking of UVB and UVA and/or reactive oxygen species damage refers to blocking of biologically relevant UVB and UVA and/or reactive oxygen species damage. Compositions of the invention may not necessarily block 100% of UVB/UVA and/or reactive oxygen species damage; equally prior art composition(s) may block UVB/UVA and/or reactive oxygen species damage, but at only insignificant levels. The invention is concerned with blocking of UVB/UVA and/or reactive oxygen species damage at biologically relevant levels. The invention is concerned with blocking of UVB/UVA and/or reactive oxygen species damage at levels relevant for application as a sunscreen. In case any further guidance is needed, this effect may be easily tested by assessing DNA photodamage - the assessment may be based on single cell gel electrophoresis (the comet assay), which detects lesion specific damage to nuclear DNA. Alternatively, or in addition, detection of total intracellular reactive oxygen species (ROS) generated by exposure to UVR may be done with established fluorescent dye markers.

### ABSORPTION

The food and drug administration (FDA) definition of "broad spectrum" for sunscreens is that 85% of a certain wavelength of UVA radiation should be absorbed. This is sometimes referred to as the "critical wavelength test". It is an advantage of the invention that biological protection is achieved with the compositions taught herein. Whether or not the compositions taught herein meet the FDA's critical wavelength test does not affect the invention described.

### SOURCES OF PALYTHINE

Palythine may be derived from any suitable source. For example, palythine may be synthesised. For example, palythine may be extracted from seaweeds, from animals such as corals, echinoderms (starfish and sea cucumbers), or vertebrates (such as fish).

Palythine is also unique in having multiple sources of the natural material. One is from animal sources. Invertebrates (e.g., corals, echinoderms) and vertebrates (fish) accumulate MAAs such as palythine, palythinol and asterina-330. The MAA's found in such animal sources tend to have a single carboxylic acid group, and are amphoteric.

Intertidal seaweeds are also a rich source of MAAs (usually shinorine and porphyra-334). MAAs found in these plant sources generally have an additional carboxylate group in their substituted imino-carbonyl residue, making them acidic.

However palythine, as well as having been isolated from animal sources, such as corals and fish, has also been found in seaweed. Indeed the South East Asian seaweed *Chondrus yendoi* produces palythine as its sole MAA product. This seaweed is therefore unique in producing the amphoteric MAA, palythine, rather than the acidic MAAs usually found in such plant materials.

This edible seaweed is collected in Japan as source of carrageenan for industrial use and food processes. It is eaten fresh in Japan during the springtime and so can be considered neither toxic nor allergenic for human use. This is a further significant advantage of palythine and the compositions such as skin-care compositions of this invention.

Most suitably palythine is extracted from seaweed. Most suitably palythine is extracted from South East Asian seaweed *Chondrus yendoi.* It is an advantage that palythine can be extracted from seaweed such as *Chondrus yendoi* in as much as 0.1% by weight.

The purification of palythine from a starting material such as seaweed is known in the art. An exemplary procedure for palythine purification is for example by a conventional aqueous, alcoholic or aqueous / alcoholic extraction process. The seaweed is first ground or cut into small pieces. The material is mixed with purified water, ethanol or with an aqueous ethanol mixture for extraction at room temperature. The solution obtained is passed down a carbon chromatography column, where the palythine is retained. The product is released from the column by further washing with aqueous methanol. The resulting extract is concentrated and purified or a solid is formed from the liquid extract, for example by drying techniques, evaporation, atomization or lyophilization.

In case any further guidance is required, further details are provided in the examples section below.

### COMPOSITIONS OF THE INVENTION

Suitably compositions of the invention comprise palythine, or salts thereof, as the sole active ingredient. By "active ingredient" it is meant protection against UVA damage, and/or protection against skin aging, and/or protection against UVB damage, and/or protection against reactive oxygen species generated by UVR.
Suitably compositions of the invention comprise palythine, or salts thereof, as the sole suncreening agent.

By "active ingredient" it is meant the biologically active sunscreening agent. Most suitably "active ingredient" means the principal substance providing sunscreen protection against UVA damage and/or UVB damage. More suitably "active ingredient" means the principal substance providing sunscreen protection against UVA damage and/or UVB damage and/or reactive oxygen species damage.

Suitably compositions of the invention comprise: a sunscreening agent consisting essentially of palythine, or salts thereof, in an amount of 0.3% to 25% by weight
Sunfilter or sunscreen compositions are regulated under European law and the amount of active ingredient by weight is limited. Suitably compositions according to the invention comply with regulation in this regard.

In another aspect, the invention provides a composition such as a skin-care composition wherein the palythine, or salts thereof, provides protection to the user against the deleterious effects of UVR. Because palythine is effective against UVA and UVB radiation and offers anti-oxidant protection, it may be used in a composition in the substantial absence of any other UV-protective filter. Thus it can be used as the predominant UV-protective active ingredient in the composition. Suitably the palythine, or salts thereof, comprises at least 50% of the active ingredients, preferably at least 75% of the active ingredients. Indeed, skin-care compositions of this invention suitably contain palythine, or salts thereof, as the sole active ingredient.

Thus suitably the compositions of the invention comprise a single active ingredient. Suitably the compositions of the invention comprise palythine, or salts thereof, as the only active ingredient.
Suitably the compositions of the invention comprise palythine, or salts thereof, as the only MAA.
Suitably the compositions of the invention do not comprise, or comprise only trace amounts of, MAAs other than palythine, or salts thereof.
Suitably the compositions of the invention do not comprise MAAs other than palythine, or salts thereof.

Suitably the compositions of the invention omit or exclude MAAs other than palythine, or salts thereof.

UVA exposure can induce collagen degrading enzymes. This can cause effects of aging such as wrinkles.
UVB exposure can induce collagen degrading enzymes. This can cause effects of aging such as wrinkles.

UVB damage can induce an immune response. UVB damage such as burning (sunburn) is an inflammatory response. UVB damage can take the form of DNA damage. DNA damage is problematic as it can predispose the subject to skin cancers.

Reactive oxygen species (ROS) can cause chemical damage such as lipid breakdown.

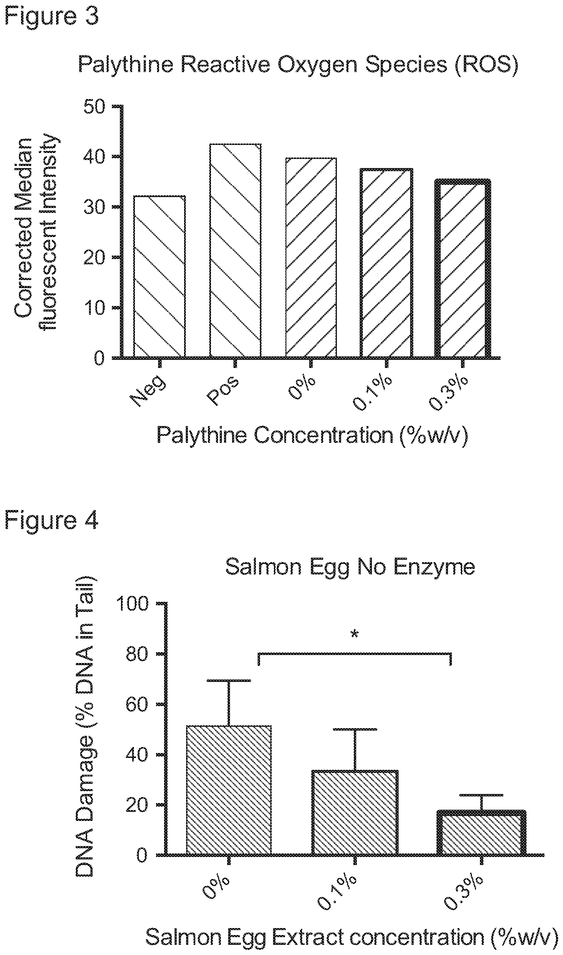

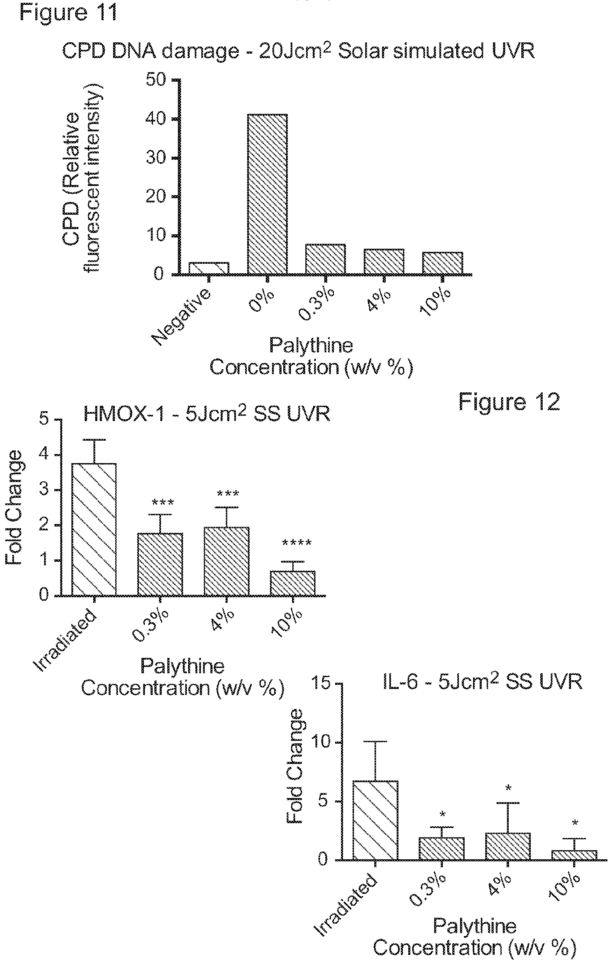

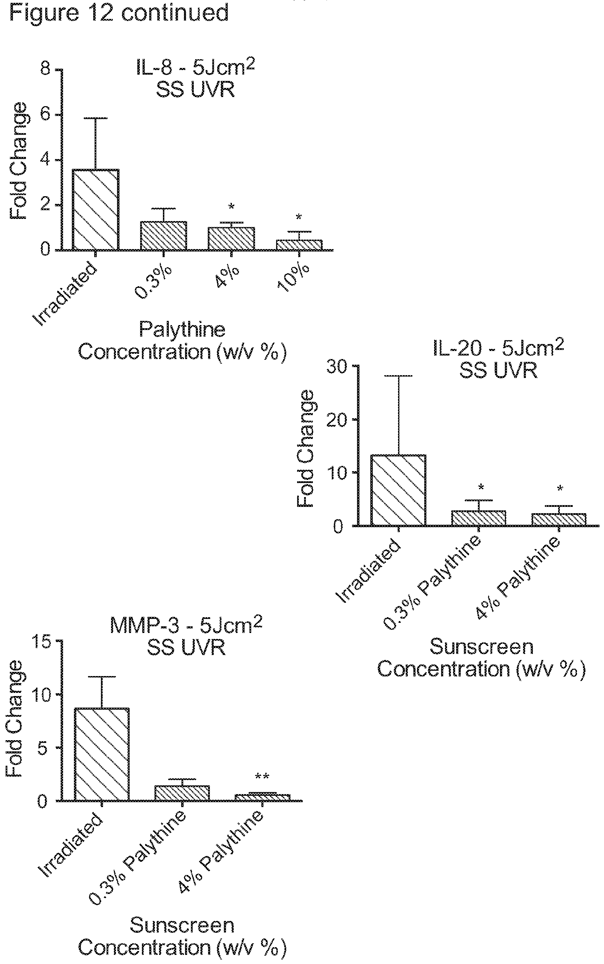

Unless otherwise apparent from the text, the term 'antioxidant' should have its normal meaning in the art. In case any guidance is needed, Figure 12 shows HMOX1 data which are indicative of antioxidant effect. In case any further guidance is needed, Figure 3 shows ROS data which are indicative of antioxidant effect.

It is an advantage of the invention that palythine, or salts thereof, is the sole active ingredient in the compositions described. An active ingredient is one which can exert an anti-aging effect; and/or one which can exert an anti-UVA damage effect; and/or one which can exert an anti-UVB damage effect; and/or which can exert an anti-reactive oxygen species damage effect. Suitably palythine, or salts thereof, provides protection against at least two of these four factors. Suitably palythine, or salts thereof, provides protection against at least three of these four factors. Suitably palythine, or salts thereof, provides protection against all four of these factors.

It is a problem with prior art compositions that they require a plurality of distinct active ingredients to obtain these effects. It is an advantage of the invention that palythine, or salts thereof, as a sole active ingredient provides each of these three effects.

It is an advantage of the invention that palythine, or salts thereof, is used at sufficiently high concentrations to achieve the biological effects described. Palythine present in trace amounts in prior art compositions has been present at such low concentrations (i.e. trace amounts or insignificant amounts) that the biological effects described are not achieved. Thus, it is a key contribution of the invention that palythine, or salts thereof, concentrations sufficient to achieve advantageous biological effects are taught.

Suitably palythine, or salts thereof, is used as a UVA protectant.

Suitably palythine, or salts thereof, is used as a UVB protectant.

Suitably palythine, or salts thereof, is used as an antioxidant.

Suitably the composition of the invention is used as a UVA protectant.

Suitably the composition of the invention is used as a UVB protectant.

Suitably the composition of the invention is used as an antioxidant.

Suitably compositions are for use on animals such as mammals, most suitably humans.

Suitably compositions are for topical application.

Suitably the composition of the invention is suitable for application to skin. Suitably compositions are for application to the skin. Suitably compositions are for application to mammalian skin, such as human skin.

Suitably compositions are for application to the external surface of the skin.

Suitably the composition of the invention is a skin-care composition.

Suitably the composition of the invention is a personal care composition.

Suitably the composition of the invention is a sun filter.

Suitably the composition of the invention is a sunscreen. When a composition is a sunscreen, suitably it comprises palythine, or salts thereof, in an amount effective to reduce DNA damage to 50% or less. If any guidance is required, suitably this may be assessed as in Example 9.

Suitably the composition of the invention is a moisturiser. When the composition is a moisturiser, suitably said composition comprises an emollient.

Suitably the composition of the invention is a moisturiser and is a sunscreen.

Suitably the composition of the invention is a skin-care composition and is a sunscreen.

Suitably the composition of the invention is a skin-care composition and is a sun filter.

Suitably the composition of the invention is a man-made composition.
Suitably the composition of the invention is an *in vitro* composition.
Suitably the composition of the invention comprises naturally occurring palythine purified/processed into a form not found in nature e.g. purified from the naturally occurring host organism such as seaweed. Thus the extracted palythine can be seen to be an isolated substance. Thus the extracted palythine can be seen to be non-naturally occurring in the form of the invention. The non-naturally-occurring formulations of the invention happen to include palythine which is a naturally occurring amino acid, but which is not naturally occurring in its purified form. The compositions of the invention can therefore be seen to be made by intervention of the hand of man. The compositions of the invention do not occur in nature.

Most suitably, palythine is purified from seaweed. The seaweed is available commercially, such as from Hokuyo-syokusan Co Ltd, Japan.

The composition of the invention may be a cream such as a cream comprising 0.3% to 25% palythine, or salts thereof. This may be manufactured according to any suitable method known in the art, such as conventional mixing at room temperature (e.g. 18-24 degrees Celsius).

Most suitably a sun filter composition should be naturally eco-compatible, non-toxic, and effective against UVA and UVB, as well as having anti-oxidant properties.

The skin-care composition of this invention may be in the form of a moisturiser, cream, lotion or spray, intended for topical application to the skin or hair to provide protection against the sun's rays or other sources of ultraviolet radiation. The active ingredient may also be included in a cosmetic/toiletry composition, for example gels such as bath gels or shower gels, shampoos optionally containing conditioning agents and/or antidandruff agents, hair conditioners, liquid soaps, creams and lotions. Such compositions may be emulsions (oil-in-water emulsions or water-in-oil emulsions).

A preferred skin-care composition of this invention is a sunscreen in the form of a cream which can be applied to the skin.

A sunscreen may be a lotion, spray, gel or other formulation such as an ointment.
A sunscreen formulation means a formulation for topical application.
A sunscreen formulation means a formulation suitable for application to skin such as human skin.

Compositions of the invention may be creams.
Compositions of the invention may be oil-in-water formulations.

Another preferred skin-care composition of this invention is an anti-aging product.

Although palythine, or salts thereof, is highly effective as the sole active ingredient, in some embodiments the compositions of this invention may contain additional inorganic or organic sun filters, preferably other MAAs. Suitably palythine, or salts thereof, is the sole active ingredient.

The skin-care composition of this invention may also include at least one cosmetically acceptable adjuvant or additive, such as a preservative, organic solvent, browning agent, antioxidant, stabilizer, emollient, silicone, alpha- hydroxy acid, demulcent, antifoaming agent, moisturizing agent, vitamin, fragrance, ionic or nonionic thickener, surfactant, filler, thickener, sequestrant, polymer, propellant, alkalinizing or acidifying agent, opacifier, fatty compound, perfume or colourant.

### PALYTHINE AMOUNTS

Unless otherwise apparent from the text, palythine, or salts thereof, amounts mentioned herein refer to final concentrations in the composition. Final concentrations in the composition refer to the composition as formulated for use, e.g. for topical application to skin such as human skin.

Suitably compositions of the invention comprise palythine, or salts thereof, at 0.3% or more.

Suitably compositions of the invention comprise palythine, or salts thereof, at 30% or less.
Suitably compositions of the invention comprise palythine, or salts thereof, at 25% or less.

Suitably compositions of the invention comprise palythine, or salts thereof, in the range 0.3% to 30%.
Suitably compositions of the invention comprise palythine, or salts thereof, in the range 0.3% to 25%.
Suitably compositions of the invention comprise palythine, or salts thereof, in the range 0.3% to 11%.
Suitably compositions of the invention comprise palythine, or salts thereof, in the range 0.3% to 10%.
Suitably compositions of the invention comprise palythine, or salts thereof, in the range 0.3% to 9%.

Suitably compositions of the invention comprise palythine, or salts thereof, in a range with a starting amount selected from the table 'start' below to 25%.
Suitably compositions of the invention comprise palythine, or salts thereof, in a range with a starting amount selected from the table 'start' below to 10%.
Suitably compositions of the invention comprise palythine, or salts thereof, in a range with a starting amount selected from the table 'start' below to 9%.

Suitably compositions of the invention comprise palythine, or salts thereof, in a range from 0.3% to an end amount selected from the table 'end' below.

Suitably compositions of the invention comprise palythine, or salts thereof, in a range with a starting amount selected from the table 'start' below to an end amount selected from the table 'end' below.

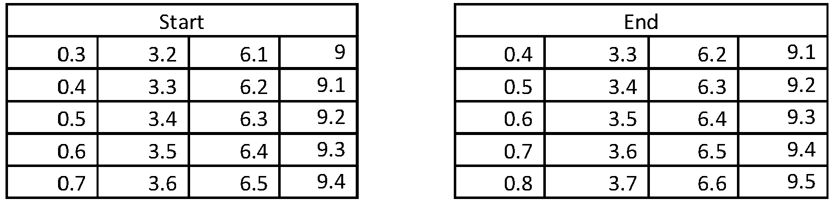

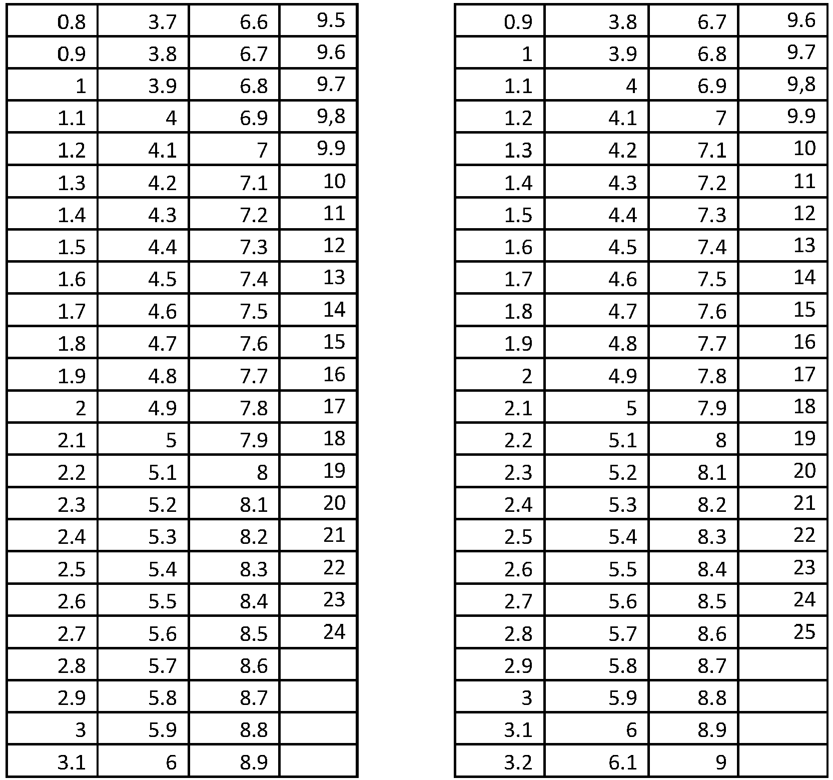

Suitably compositions of the invention comprise palythine, or salts thereof, in an amount selected from either the table 'start' above or the table 'end' above.

For the avoidance of doubt, 'start' should always be lower than 'end'.

The compositions of this invention may suitably contain from 0.3 to 30% by weight of palythine, or salts thereof, suitably from 0.3 to 25% by weight of palythine, or salts thereof, suitably from 0.3 to 10% by weight of palythine, or salts thereof, more suitably from 0.3 to 9% by weight of palythine, or salts thereof, suitably 0.5 to 9% by weight of palythine, or salts thereof, and most suitably 3 to 5% by weight of palythine, or salts thereof.

**Table 1 - Palythine Concentrations for SPF values**

| **Palythine (=UV filter+antioxidant)** | **SPF** |
|---|---|
| **Concentration (% w/v)** | |
| 0.3-1 | 5 |
| 1-5 | 15 |
| 5-15 | 30 |
| 15-20 | 50 |
| 20-30 | 50+ |

### Supplier Information:

Hokuyo-syokusan Co Ltd
194 Aza-minato-cho, Oshidomari, Rishirifuji-cho, Rishiri-gun, Hokkaido 097-0101 JAPAN
http://www.hoshitako.co.jp/index.html
FAX: +81-11-380-4102

In the below examples of compositions, after addition of the desired amount of palythine (=UV filter+antioxidant), unless otherwise specified the balance of the components should be made up to 100% with solvent/diluent such as water. By way of illustration, each example below adds up to 70% and leaves 30% for (palythine (=UV filter+antioxidant) + solvent/diluent such as water), therefore where less than 30% palythine is used, the balance should be made up to 100% with water.

### Examples of Sunscreen Spray Compositions

**Contains the following:**

| **Component** | | **% w/v** | | | |
|---|---|---|---|---|---|
| Solvent | | 58.2 | | | |
| Emulsifier | | 2.5 | | | |
| Emolient | | 4 | | | |
| Preservative | | 0.6 | | | |
| Rheology Modifier | | 1.5 | | | |
| pH Adjuster | | 2.7 | | | |
| Non-greasy agent | | 0.3 | | | |
| Chelating agent | | 0.2 | | | |
| UV filter + Antioxidant | | See Table 1 | | | |

| | | **Useful** | **Better** | **Best** | **Specific Example** |
|---|---|---|---|---|---|
| **Function** | **Chemical Name** | **% w/v** | | | |
| Solvent | Water | 40-80 | 50-70 | 55-65 | 58.2 |
| Emulsifier | Methyl Glucose Dioleate | 0.1 - 10 | 1-5 | 1.5-4 | 2.5 |
| Emolient | Cetyl Ethylhexanoate | 0.1 - 10 | 1-5 | 1.5-4 | 2 |
| Emolient | Isodecyl Neopentanoate | 0.1 - 5 | 0.5-5 | 0.75-2.5 | 1 |
| Emolient | Cocoyl Adipic Acid | 0.1 - 5 | 0.5-5 | 0.75-2.5 | 1 |
| Preservative | Potassium Sorbate | 0.001-5 | 0.01-1 | 0.05-0.5 | 0.1 |
| Preservative | Phenoxyethanol | 0.001-5 | 0.01-1 | 0.1-1 | 0.5 |
| Rheology modifier | Acrylates Copolymer | 0.001-5 | 0.5-5 | 0.75-2.5 | 1 |
| Rheology modifier | Beheneth-25 Methacrylate Copolymer | 0.001-5 | 0.01-1 | 0.1-1 | 0.5 |
| pH Adjuster | Sodium Hydroxide | 0.1 - 10 | 1-5 | 1.5-4.5 | 2.7 |
| Non-greasey agent | Nylon-12 | 0.001-5 | 0.01-1 | 0.1-1 | 0.3 |
| Chelating agent | EDTA | 0.001-5 | 0.01-1 | 0.1-1 | 0.2 |
| UV Filer + Antioxidant | Palythine | See Table 1 | See Table 1 | See Table 1 | See Table 1 |

### Method:

1. Dissolve Palythine, EDTA and Potassium sorbate in water.
2. Add Acrylates Copolymer and Beheneth-25 Methacrylate Copolymer
3. Adjust with sodium hydroxide to pH 6.9-7.3
4. Heat the mixture to 70-75°C
5. Seperately mix Methyl Glucose Dioleate, Cetyl Ethylhexanoate, Isodecyl Neopentanoate, Cocoyl Adipic Acid and Phenoxyethanol.
6. Add the two mixtures together along with Nylon-12
7. Allow to cool

### Examples of Sunscreen Lotion compositions

**Contains:**

| **Component** | | **% w/v** | | | |
|---|---|---|---|---|---|
| Solvent | | 55.25 | | | |
| Emolient | | 7 | | | |
| pH Adjuster | | 0.5 | | | |
| Preservative | | 0.5 | | | |
| Rheology Modifier | | 0.35 | | | |
| Emulsifier | | 0.2 | | | |
| Surfactant | | 0.1 | | | |
| Humectant | | 6 | | | |
| Chelating agent | | 0.1 | | | |
| UV Filter + Antioxidant | | See Table 1 | | | |

| | | **Useful** | **Better** | **Best** | **Specific Example** |
|---|---|---|---|---|---|
| **Function** | **Chemical Name** | **% w/v** | | | |
| Solvent | Water | 40-80 | 50-70 | 55-65 | 55.25 |
| Emoilient | C12-15 Alkyl Benzoate | 0.1 - 10 | 1-5 | 2-4 | 3 |
| Emolient | Diisopropyl Sebacate | 0.1-10 | 1-8 | 2-6 | 4 |
| pH Adjuster | Sodium Hydroxide | 0.001-5 | 0.01-1 | 0.1-1 | 0.5 |
| Preservative | Phenoxyethanol | 0.001-5 | 0.01-1 | 0.1-1 | 0.5 |
| Rheology Modifier | Xanthan Gum | 0.001-5 | 0.01-1 | 0.05-0.5 | 0.1 |
| Rheology Modifier | Carbomer | 0.001-5 | 0.01-1 | 0.1-1 | 0.25 |
| Emulsifier | C10-30 Alkyl Acrylate Crosspolymer | 0.001-5 | 0.01-1 | 0.1-1 | 0.2 |
| Surfactant | Trideceth-6 | 0.001-5 | 0.01-1 | 0.05-0.5 | 0.1 |
| Humectant | Glycerin | 0.1-12 | 2-10 | 04-08 | 6 |
| Chelating agent | EDTA | 0.001-5 | 0.01-1 | 0.05-0.5 | 0.1 |
| UV Filter + Antioxidant | Palythine | See Table 1 | See Table 1 | See Table 1 | See Table 1 |

### Method:

1. Dissolve the Glycerin, Trideceth-6, palyhine and Disodium EDTA in the water.
2. Add the Xanthan Gum, C10-30 Alkyl Acrylate Crosspolymer and Carbomer in order.
3. Heat the mixture to 70-75°C
4. Seperately mix Diisopropyl Sebacate and C12-15 Alkyl Benzoate and heat to 70-75°C.
5. Mix the two solutions and allow to cool to 50°C.
6. Adjust with sodium hydroxide to pH 6.9-7.3
7. Cool to room temperature and add Phenoxyethanol.

### Examples of Sun Stick Compositions

| **Component** | **% w/v** |
|---|---|
| Solvent | 17.5 |
| Emollient | 13 |
| Binding Agent | 3 |
| Emulsifier | 5.5 |
| Structurant | 19 |
| Stabilizer | 10 |
| Dispersant | 2 |
| UV filter + Antioxidant | See Table 1 |

| | | **Useful** | **Better** | **Best** | **Specific Example** |
|---|---|---|---|---|---|
| **Function** | **Chemical Name** | **% w/v** | | | |
| Solvent | Diisopropyl Adipate | 5-25 | 10-20 | 12.5-17.5 | 17.5 |
| Emollient | Isostearyl Isosterate | 1-30 | 5-20 | 10-15 | 13 |
| Binding Agent | Isostearyl Hydroxystearate | 0.1 - 10 | 1-5 | 2-4 | 3 |
| Emulsifier | Cetearyl Alcohol Cetearah 20 | 0.1-10 | 1-8 | 3-7 | 5.5 |
| Structurant | Microcrystaline Wax | 2-16 | 4-14 | 6-10 | 8 |
| Structurant | Polyethylene | 0.1-10 | 1-8 | 3-7 | 5 |
| Structurant | Ozokerite Wax | 0.1 - 10 | 1-5 | 2-4 | 3 |
| Structurant | Rhus Succedanea Fruit Wax | 0.1 - 10 | 1-5 | 2-4 | 3 |
| Stabilizer | Butyloctyl Salicylate | 1-20 | 5-15 | 8-12 | 10 |
| Dispersant | Methyl Glucose Dioleate | 0.1 - 10 | 1-5 | 1.5-4 | 2 |
| UV filter + Antioxidant | Palythine | See Table 1 | See Table 1 | See Table 1 | See Table 1 |

### Method:

1. Mix Diisopropyl Adipate, Isostearyl Isosterate, Isostearyl Hydroxystearate, Methyl Glucose Dioleate, Butyloctyl Salicylate and palythine
2. Add remaining ingredients and heat to 80-95°C.
3. Pour into mold and cool

### Examples of Sunscreen Cream Compositions

| **Component** | **% w/v** |
|---|---|
| Diluent | 32.7 |
| Chelating Agent | 0.2 |
| Emolient | 16.5 |
| Emulsifier | 6 |
| Stabilizer | 1.3 |
| pH Adjuster | 2.8 |
| Humectant | 10 |
| Preservative | 0.5 |
| UV Filter + Antioxidant | See Table 1 |

| | | **Useful** | **Better** | **Best** | **Specific Example** |
|---|---|---|---|---|---|
| **Function** | **Chemical Name** | **% w/v** | | | |
| Diluent | Water | 20-50 | 25-45 | 30-40 | 32.7 |
| Chelating Agent | EDTA | 0.001-5 | 0.01-1 | 0.1-1 | 0.2 |
| Emolient | Cocyl Adipic Acid | 0.1 - 10 | 1-5 | 1.5-4 | 2 |
| Emolient | Cyclopentasiloxane Dimethicone crosspolymer | 0.1 - 10 | 1-5 | 2-4 | 3 |
| Emolient | Cyclopentasiloxane | 0.1 - 10 | 1-5 | 2-4 | 3 |
| Emolient | Diisopropyl Sebacate | 2-16 | 4-14 | 6-10 | 8.5 |
| Emulsifier | Methyl Glucose | 0.1 - 10 | 1-5 | 1.5-4 | 2 |
| | Sesquisterate | | | | |
| Emulsifier | PEG-20 Methyl Glucose Sesquisterate | 0.1 - 10 | 1-5 | 1.5-4 | 2 |
| Emulsifier | Potassium Cetyl Phosphate | 0.1 - 10 | 1-5 | 1.5-4 | 2 |
| Stabilizer | Xantham Gum | 0.001-5 | 0.01-1 | 0.1-1 | 0.3 |
| Stabilizer | Acrylates copolymer | 0.1-5 | 0.5-5 | 0.75-2.5 | 1 |
| pH Adjuster | Sodium Hydroxide | 0.1 - 10 | 1-5 | 2-4 | 2.8 |
| Humectant | Glycerin | 0.1-10 | 1-8 | 3-7 | 5 |
| Humencant | Butylene Glycol | 0.1-10 | 1-8 | 3-7 | 5 |
| Preservative | Phenoxyethanol | 0.001-5 | 0.01-1 | 0.1-1 | 0.5 |
| UV Filter + Antioxidant | Palythine | See Table 1 | See Table 1 | See Table 1 | See Table 1 |

### Method:

1. Dissolve Glycerin, Butylene Glycol, Palythine and EDTA in water
2. Adjust pH to pH 7.0-7.3
3. Add in order Acrylates copolymer, Xantham gum and Potassium Cetyl Phosphate.
4. Heat to 70-75°C.
5. Mix separately the remaining ingreidents with the exception of Cyclopentasiloxane Dimethicone crosspolymer Cyclopentasiloxane and Phenoxyethanol at 70-75°C.
6. Add two mixtures together and cool to 50°C.
7. Add Cyclopentasiloxane Dimethicone crosspolymer Cyclopentasiloxane and Phenoxyethanol

**Suppliers**

| **Ingredient** | **Supplier** | **Order Code/Name** |
|---|---|---|
| Acrylates copolymer | Sigma-Aldrich | 1265424 |
| Beheneth-25 Methacrylate Copolymer | Lubrizol | Novethix™ L-10 Polymer |
| Butylene Glycol | Sigma-Aldrich | S359173 |
| Butyloctyl Salicylate | Hallstar | 12231 |
| C10-30 Alkyl Acrylate Crosspolymer | Lubrizol | Carbopol® Ultrez 20 Polymer |
| C12-15 Alkyl Benzoate | Parchem | 68411-27-8 |
| Carbomer | Surfachem | Carbopol® 980 |
| Cetearyl Alcohol Cetearah 20 | Hallstar | 1H064 |
| Cetyl Ethylhexanoate | Parchem | 90411-68-0 |
| Cocyl Adipic Acid | Sigma-Aldrich | 9582 |
| Cyclopentasiloxane | SpecialChem | 541-02-6 |
| Cyclopentasiloxane Dimethicone crosspolymer | SpecialChem | |
| Diisopropyl Adipate | Sigma-Aldrich | S563889 |
| Diisopropyl Sebacate | Parchem | 2042106 |
| EDTA | Sigma-Aldrich | EDS |
| Glycerin | Sigma-Aldrich | G5516 |
| Isodecyl Neopentanoate | Lubrizol | Schercemol™ 105 Ester |
| Isostearyl Hydroxystearate | Lubrizol | Schercemol™ SHS |
| Isostearyl Isosterate | Lubrizol | Schercemol™ 1818 Ester |
| Methyl Glucose Dioleate | Lubrizol | Glucamate™ DOE-120 Thickener |
| Methyl Glucose Sesquisterate | Lubrizol | Glucamate™ SSE-20 Emulsifier |
| Microcrystaline Wax | | |
| Nylon-12 | Sigma-Aldrich | 181161 |
| Ozokerite Wax | | |
| Palythine | Hokuyo-syokusan Co Ltd | Palythine |
| PEG-20 Methyl Glucose Sesquisterate | Lubrizol | Glucamate™ SSE-20 Emulsifier |
| Phenoxyethanol | Sigma-Aldrich | P1126 |
| Polyethylene | Sigma-Aldrich | 81219 |
| Potassium Cetyl Phosphate | Parchem | 19035-79-1 |
| Potassium Sorbate | Sigma-Aldrich | 47848 |
| Rhus Succedanea Fruit Wax | Parchem | 8001-39-6 |
| Sodium Hydroxide | Sigma-Aldrich | 415413 |
| Trideceth-6 | Parchem | Trideceth-6 |
| Water | Sigma-Aldrich | W4502 |
| Xanthan Gum | Sigma-Aldrich | G1253 |

### ADVANTAGES

It is a problem in the art that prior art sunscreen products are very complex. Prior art sunscreen products typically have multiple active ingredients. It is an advantage of the invention that the compositions require only a single active ingredient i.e. palythine, or salts thereof.

It is an advantage of the invention that palythine, or salts thereof, is able to biodegrade into eco-compatible metabolites.

It is an advantage of the invention that the compositions are non-toxic.

It is an advantage of the invention that the active ingredient palythine, or salts thereof, is non-toxic at the concentrations taught herein.

It is an advantage of the invention that palythine, or salts thereof, is water soluble.

It is an advantage of the invention that compositions such as skin-care compositions are described using palythine, or salts thereof, which is a naturally occurring compound that is eco-compatible. Although some natural products have been used as sun filters, most such products are protective only against UVA radiation. A second filter must be added in order to provide UVB protection. Furthermore, often a separate anti-oxidant is incorporated, whereas palythine advantageously also provides this property.

It is advantageous to have as few active ingredients as possible in a composition such as a skin-care composition, in order to reduce possible interaction between the ingredients and to reduce potential toxicity and/or allergy issues. In addition, formulation can be simplified with fewer active ingredients, as well as costs reduced.

Given that the majority of known commercially available sunscreens are non-biodegradable synthetic compounds that are also pollutants and toxic to aquatic life, man-made formulations incorporating naturally occurring MAAs may be an ecologically friendly future option for human sunscreen applications.

Known 'plant-type' MAA mixtures isolated from seaweed are available for commercial use in human cosmetic applications, yet there are only two published photoprotection studies on mammalian skin/cells. One study has shown these MAA mixtures inhibited acute photodamage in mouse skin *in vivo* and the other study showed uptake by a human skin carcinoma cell line, strongly suggesting that MAAs could be used as sunscreens for human applications. However, these 'plant-type' MAA mixtures only absorb in the UVA (315-400nm) region of the UVR spectrum and clear evidence for effective photoprotection has not been demonstrated.

Marine animals accumulate "non-acidic" MAAs as mixtures of typically palythine, palythinol and asterina-330. Our data shows that these "non-acidic" MAAs as naturally occurring mixtures offer protection from photomolecular damage that may lead to skin cancer and photoageing. Palythine is a unique MAA because it absorbs in both the UVA and UVB regions of the UVR. We have now shown the ability of palythine alone to protect against the formation of ROS, 8-oxoGua and CPD. Thus we teach for the first time that palythine is an antioxidant and can also offer protection against the DNA damaging effects of UVA and UVB exposure.
It is an advantage of the invention that palythine's antioxidant effects can be utilised in compositions such as sunscreen and/or skincare compositions. In the art, additional ingredients are typically required to obtain such antioxidant effects. In some embodiments, compositions of the invention may be applied post-irradiation for beneficial antioxidant effects. It is an advantage of the invention that a composition of the invention applied as a sunscreen/filter will then be present on the subject's skin immediately post-irradiation, thereby supplying antioxidant effect immediately post-irradiation.

The palythine used in this study was isolated from *Chondrus yendoi* collected in Japan. In Japan, this seaweed is eaten fresh during the springtime so is nontoxic or allergenic. This is a great advantage in sunscreen compositions of the invention because such accumulation is not considered desirable with conventional organic based sunscreens.

Palythine, or salts thereof, absorbs both in the UVA and UVB regions of the UVR spectrum and prevents formation of reactive oxygen species which is unique for an MAA.

As explained in the Background above, FR1250912 does not provide any enabling disclosure of a sunscreen composition comprising palythine. FR1250912 uses a mixture of 5 MAAs from Polysiphonia lanosa for UVA/B protection and the compound astaxanthin from Haematococcus pluvialis as an antioxidant. Our composition uses just palythine at 0.3% lower limit UVA/B protection and antioxidant activity. The only information available in FR1250912 regarding the MAAs used seems to be at page 2 of the 'documents considered important' (page 29 of FR1250912) which shows a reference to a study by Karsten et al., 1998. This is the only published scientific paper describing MAAs in Polysiphonia known to the inventors. Nowhere in this document is there any disclosure of any sunscreen composition. Two species of Polysiphonia were studied by Karsten et al., 1998: Polysiphonia artica which was found to contain only 0.462 mg palythine per gram dry weight of the alga and, Polysiphonia elongate where palythine could not be detected. Even extrapolating from FR1250912 and producing a composition using 10% MAA mixture from other sources (which FR1250912 fails to properly disclose) results only in a maximum hypothetical palythine concentration of 0.00462% (using MAA mixture disclosed by Karsten et al 1998 at the level of 10% disclosed in FR1250912 = palythine at 10% of the maximum concentration in Karsten 1998 of 0.462 mg/g=0.00462%). Thus, even a thorough review of the literature by the inventors attempting to fill in gaps left by FR1250912 results in a composition containing no more than 0.00462% by weight palythine. This analysis is made by the inventors and does not amount to any acceptance that a skilled person would make that combination, even though making that combination does not lead to the invention.

The composition in WO2007/026037 is for an industrial antioxidant using a mixture of 2 MAAs. Our composition is a sunscreen (and/or moisturiser), each with UVA/B protection and antioxidant activity for human use using only palythine, or salts thereof, as the single active ingredient (single MAA). At 0.0008 % in WO2007/026037, palythine alone would not have effective antioxidant activity (based on our data). Hence in WO2007/026037 the antioxidant activity can only be due to asterina-330 alone or asterina-330 + palythine (to the best of the inventors' knowledge, the antioxidant activity of asterina-330 alone has never been disclosed in the literature).

### MANUFACTURE

Suitably the composition of the invention is manufactured according to any well known method in the art for making sunscreens, for example sunscreens in the form of creams such as topical creams.

In particular, suitably the composition of the invention is manufactured as described in WO2013/014584. Certain elements and/or methods below are taken from WO2013/014584 for illustrative purposes.

Suitably the composition of the invention is a sunscreen composition comprising:
- palythine, or salts thereof,
- one or more antioxidants selected from tocopheryl acetate, tocopherol, retinol, polyphenolic compounds extracted from green tea,
and free from preservatives belonging to the paraben family.

Suitably the composition of the invention is a sunscreen composition comprising:
- a sunscreen agent consisting essentially of palythine, or salts thereof,
- one or more antioxidants selected from tocopheryl acetate, tocopherol, retinol, polyphenolic compounds extracted from green tea,
and free from preservatives belonging to the paraben family.

The phrase "eco-compatible" such as "eco-compatible with the marine ecosystem" of the coral reefs means a product causing no harm or alteration to the marine organisms or to the communities or habitats as a whole. An eco-compatible product is therefore usable without restrictions, as it protects the health of the environment and biodiversity. Known tests may be followed to check the health conditions of marine organisms exposed to the various chemical components if necessary in order to select/confirm that the components are absolutely innocuous.

The composition of the present invention may comprise:
- palythine, or salts thereof;
- one or more antioxidants selected from tocopheryl acetate, tocopherol, retinol, polyphenolic compounds extracted from green tea, and which are free from preservatives belonging to the paraben family.

In the present description, by "parabens" are meant the esters of 4-hydroxybenzoic acid used as preservatives in the cosmetics industry. The most common parabens are methylparaben, ethylparaben, propylparaben and butylparaben.

An example of polyphenols extracted from green tea is polyphenone-6o (CAS N° 138988-88-2) commercially available from Sigma.

In some embodiments, the compositions of the present invention may comprise one or more fragrances, suitably eco-compatible with the marine environment, selected from the group consisting of fragrances of orange, lavender, grapefruit, guava and coconut.

Examples of fragrances suitable for the present invention are the fragrances commercially available from FAROTTI SRL company, such as guava and coconut, natural lavender, natural grapefruit, preferably in concentrations equal to or lower than 0.3% of the product.

In order to increase the shelf life times, in an embodiment the compositions may comprise a preservative such as sorbic acid. Sorbic acid is not harmful to marine life in conjunction with other commonly used compounds.

The compositions may be prepared in different cosmetic forms, such as, e.g., cream, lotion, ointment, spray, lipstick.

The compositions may be cosmetic compositions or cosmetic formulations. Suitably cosmetic compositions are made up so as to fall within the category "cosmetic product", as defined in the EU Cosmetics Directive (76/768/EEC). This category has borders with a range of product categories, including medicinal products, biocides and medical devices. The EU Cosmetics Directive defines a cosmetic product as:
"... *any substance or preparation intended to be placed in contact with the various external parts of the human body (epidermis, hair system, nails, lips and external genital organs) or with the teeth and the mucous membranes of the oral cavity with a view exclusively or mainly to cleaning them, perfuming them, changing their appearance and or*/*correcting body odours and*/ *or protecting them or keeping them in good condition. "*

The palythine, or salts thereof, antioxidants, fragrances and preservatives may be mixed with different carriers suitable for the determined cosmetic form that is to be manufactured.

Suitably, carriers having an emollient function will be used, for example selected from the group consisting of propylene glycol dicaprylate/dicaprate, olive oil, and beeswax.

Propylene glycol dicaprylate/dicaprate (CAS number: 68583-51 -7) is a derivative of propylene glycol, and is a diester of propylene glycol and fatty acids, In particular, it is a mixture of propylene glycol dicaprylate and propylene glycol dicaprate. This product is commercially available under the name Labrafac.

Beeswax (CAS number:8012-89-3) suitable for the present invention is e.g. commercially available under the name Apifil.

Thus suitably the composition of the invention is a sunscreen composition comprising:

| Component |
|---|
| emulsifier |
| emollient |
| carrier |
| Active (palythine) 0.3 to 25% (suitably by weight) |
| water |
| preservative |
| optional Fragrance |

More suitably the composition of the invention is a sunscreen composition comprising:

| Component |
|---|
| emulsifier |
| emollient |
| carrier |
| optional antioxidant |
| Active (palythine) 0.3 to 25% (suitably by weight) |
| water |
| optional gelling agent |
| preservative |
| optional Fragrance |

More suitably the composition of the invention is a sunscreen composition comprising:

| Component |
|---|
| emulsifier |
| emollient |
| carrier |
| antioxidant |
| Active (palythine) 0.3 to 25% (suitably by weight) |
| water |
| gelling agent |
| preservative |
| optional Fragrance |

More suitably the composition of the invention is a sunscreen composition comprising:

| Component |
|---|
| emulsifier |
| emollient |
| carrier |
| antioxidant |
| Active (palythine) 0.3 to 25% (suitably by weight) |
| water |
| gelling agent |
| preservative/ stabiliser |
| preservative |
| emollient |
| preservative/ alkali |
| Fragrance |

The antioxidants tocopheryl acetate and tocopherol and the fragrance of guava and coconut are compounds having a greater eco-compatibility with corals. Preferably, the compositions of the present invention will therefore comprise tocopheryl acetate or tocopherol and optionally the fragrance of guava and coconut.

The some compounds commonly used in sunscreens proved harmful to these environments, compounds such as, e.g.:
- preservatives belonging to the paraben family
- ascorbic acid derivatives;
- UV filters selected from bis ethylhexyloxyphenol methoxyphenyl triazine (BEMT), cinnamates, benzophenones, camphor derivatives, titanium oxide;
- fragrances selected from melaleuca essential oil, ylang ylang flower extracts, *Bambousa arundinacea* extracts, Karite;
- carriers selected from animal-derived fats;
- Argan oil.
Suitably these ingredients are omitted from the compositions of the invention. Suitably the compositions of the invention do not comprise said compounds.

In one embodiment, the sunscreen compositions comprise:
- palythine, or salts thereof;
- one or more antioxidants selected from tocopheryl acetate, tocopherol, retinol, polyphenolic compounds extracted from green tea;
- one or more carriers selected from propylene glycol dicaprylate/dicaprate, olive oil, beeswax
and are free from:
- preservatives belonging to the paraben family or ascorbic acid derivatives;
- UV filters selected from bis ethylhexyloxyphenol methoxyphenyl triazine, cinnamates, benzophenones, camphor derivatives, titanium oxide;
- fragrances selected from melaleuca essential oil, ylang ylang flower extracts, *Bambousa arundinacea* extracts, Karite;
- carriers selected from animal-derived fats and argan oil.

In an embodiment with a very high eco-compatibility, they will further comprise sorbic acid and/or one or more fragrances selected from orange, lavender, grapefruit, guava and coconut.

In a further highly eco-compatible embodiment the compositions consist essentially of:
- palythine, or salts thereof;
- one or more antioxidants selected from tocopheryl acetate, tocopherol, retinol, polyphenolic compounds extracted from green tea;
- one or more carriers selected from propylene glycol dicaprylate/dicaprate, olive oil, beeswax;
- water.

In a further highly eco-compatible embodiment, the compositions are essentially consisting of:
- palythine, or salts thereof;
- one or more antioxidants selected from tocopheryl acetate, tocopherol, retinol, polyphenolic compounds extracted from green tea;
- sorbic acid;
- one or more carriers selected from propylene glycol dicaprylate/dicaprate, olive oil, beeswax;
- water.

In a further embodiment, the compositions are essentially consisting of:
- palythine, or salts thereof;
- tocopheryl acetate and/or tocopherol;
- sorbic acid;
- one or more carriers selected from propylene glycol dicaprylate/dicaprate, olive oil, beeswax;
- water;
- one or more fragrances selected from orange, lavender, grapefruit, guava and coconut.

In a further embodiment, the compositions are essentially consisting of:
- palythine, or salts thereof;
- tocopheryl acetate and/or tocopherol;
- sorbic acid;
- one or more carriers selected from propylene glycol dicaprylate/dicaprate, olive oil, beeswax;
- water;
- one or more fragrances selected from orange, lavender, grapefruit, guava and coconut;
- hydroxyethyl cellulose and/or EDTA and/or sodium hydroxide.

In a further embodiment, the compositions are essentially consisting of:
- palythine, or salts thereof;
- tocopheryl acetate;
- sorbic acid;
- propylene glycol dicaprylate/dicaprate, olive oil and beeswax;
- water;
- hydroxyethyl cellulose;
- EDTA;
- sodium hydroxide.

All embodiments listed above have the advantage of eco-compatibility with the coral reef and (with no need of addition of other compounds potentially harmful for the marine ecosystem) have a high photostability and prolonged storage in a suitable cosmetic formulation. In the compositions of the present invention, the palythine, or salts thereof, is suitably in a percentage by weight in the range 0.3% to 25% by weight of the composition.

### OPTIONAL COMPONENTS

The composition of the invention may optionally comprise a further UVA filter.

The composition of the invention may optionally comprise one or more further UV filters, for example selected from 2,2'-methylene-bis-(6-(2H-benzotriazole-2-yl)-4-(1,1,3,3-tetramethylbutyl)-phenol (lUPAC name for MBBT), 2-[4-(diethylamine)-2-hydroxybenzoyljhexyl benzoate (lUPAC name for DHHB), ethylhexyl triazone (lUPAC name for EHT).

The composition of the invention may optionally comprise one or more further UV filters selected from 2,2'-methylene-bis-(6-(2H-benzotriazole-2-yl)-4-(1,1,3,3-tetramethylbutyl)-phenol, 2-[4-(diethylamine)-2-hydroxybenzoyljhexyl benzoate, ethylhexyl triazone.

Optionally the composition may comprise perfume.

Optionally the composition may comprise insect repellent.

Optionally the composition may comprise hydration agents.

Optionally the composition may comprise silk worm extract.

### FURTHER APPLICATIONS

The invention also relates to a method for reducing ultraviolet light damage to a skin or hair surface, preferably human skin or hair, which comprises applying to the skin or hair surface an effective amount of palythine, or salts thereof. The palythine, or salts thereof, may be applied in the absence of any other UV protective filter, preferably formulated in a composition of this invention.
palythine is absorbed by human cells, which suggests that it is retained on the skin. Thus, it is advantageous to make repeated applications of the compositions to the skin to build and maintain long-term protection.

Amphoteric MAAs, such as palythine, are known to accumulate in UVR/light sensitive tissues (including the skin, ocular lenses, and eggs) of fish. Thus, the skin-care compositions of this invention may also be in a form adapted for oral administration, for example as an ingestible capsule, or as a food supplement. Such compositions would have only low systemic SPF protection, but would be valuable against the accumulative effects of long-term UVR- photodamage.

A further type of composition of the invention is adapted for application to the human eye. This would be particularly protective against cataract formation, another known effect of long-term sun exposure.

The invention is now described by way of numbered paragraphs:
(i) In another aspect the invention provides a skin-care composition comprising palythine as defined by Formula 1, or salts thereof, wherein the palythine, or salts thereof, provides protection to the user against the deleterious effects of UVR.
(ii) A skin-care composition as described in paragraph (i), in the substantial absence of any other UVR-protective filter.
(iii) A skin-care composition as described in paragraph (i), wherein palythine is the predominant UVR-protective active ingredient.
(iv) A skin-care composition as described in paragraph (iii), wherein palythine comprises at least 50% of the active ingredients.
(v) A skin-care composition as described in paragraph (iv), wherein palythine is the sole active ingredient.
(vi) A skin-care composition as described in any of paragraphs (i) to (v), in the form of an anti-aging product.
(vii) A skin-care composition as described in any of paragraphs (i) to (v), in the form of a sunscreen.
(viii) A skin-care composition as described in any of paragraphs (i) to (vii), which is adapted for use topically on the skin.
(ix) A skin-care composition as described in any of paragraphs (i) to (vii), which is adapted for oral use.
(x) A skin-care composition as described in any of paragraphs (i) to (vii), which is adapted for application to the human eye.
(xi) A skin-care composition as described in any of paragraphs (i) to (x), wherein palythine comprises from 0.3% to 25% by weight of the composition, suitably from 0.3% to 9% by weight of the composition.
(xii) A skin-care composition as described in any of paragraphs (i) to (xi), wherein palythine comprises from 3% to 5% by weight of the composition.
(xiii) A method for reducing UVR damage to a human or animal, which method comprises applying to the human or animal skin or hair surface, an amount of palythine sufficient to protect against UVA radiation, UVB radiation and exhibit effective anti-oxidant properties.
(xiv) A method as described in paragraph (xiii), for reducing ultraviolet light damage to a human.
(xv) A method as described in paragraph (xiii) or (xiv), wherein palythine, or salts thereof, is applied to the human or animal skin or hair in the substantial absence of any other UV protective filter.
(xvi) The use of palythine, as defined by Formula 1 herein, or salts thereof, for the protection of a human or animal against the deleterious effects of ultraviolet radiation.
(xvii) The use of palythine, or salts thereof, as a sun filter.

### ISOMERS AND SALTS

Certain compounds may exist in one or more particular geometric, optical, enantiomeric, diasteriomeric, epimeric, atropic, stereoisomeric, tautomeric, conformational, or anomeric forms, including but not limited to, cis- and trans-forms; E- and Z-forms; c-, t-, and r- forms; endo- and exo-forms; R-, S-, and meso-forms; D- and L-forms; d- and l- forms; (+) and (-) forms; keto-, enol-, and enolate-forms; syn- and anti-forms; synclinal- and anticlinal-forms; alpha- and beta-forms; axial and equatorial forms; boat-, chair-, twist-, envelope-, and halfchair-forms; and combinations thereof, hereinafter collectively referred to as "isomers" (or "isomeric forms").

Note that, except as discussed below for tautomeric forms, specifically excluded from the term "isomers", as used herein, are structural (or constitutional) isomers (i.e. isomers which differ in the connections between atoms rather than merely by the position of atoms in space). For example, a reference to a methoxy group, -OCH₃, is not to be construed as a reference to its structural isomer, a hydroxymethyl group,-CH₂OH.

A reference to a class of structures may well include structurally isomeric forms falling within that class (e.g. C₁₋₆ alkyl includes n-propyl and iso-propyl; butyl includes n-, iso-, sec-, and tert-butyl; methoxyphenyl includes ortho-, meta-, and para-methoxyphenyl).

The above exclusion does not apply to tautomeric forms, for example, keto-, enol-, and enolate-forms, as in, for example, the following tautomeric pairs: keto/enol, imine/enamine, amide/imino alcohol, amidine/amidine, nitroso/oxime, thioketone/enethiol, N-nitroso/hyroxyazo, and nitro/aci-nitro.

Note that specifically included in the term "isomer" are compounds with one or more isotopic substitutions. For example, H may be in any isotopic form, including ¹H, ²H (D), and ³H (T); C may be in any isotopic form, including ¹²C, ¹³C, and ¹⁴C; O may be in any isotopic form, including ¹⁶O and ¹⁸O; and the like.

Unless otherwise specified, a reference to a particular compound includes all such isomeric forms, including (wholly or partially) racemic and other mixtures thereof.

Methods for the preparation (e.g. asymmetric synthesis) and separation (e.g. fractional crystallisation and chromatographic means) of such isomeric forms are either known in the art or are readily obtained by adapting the methods taught herein, or known methods, in a known manner.

The invention includes compounds of Formula 1, which include compounds specifically named above, and salts thereof. Suitably, the salts of compounds of Formula 1 are pharmaceutically acceptable salts. These salts include nontoxic acid addition salts (including di-acids) and base salts.

If the compound is cationic, or has a functional group which may be cationic (e.g. -NH2 may be -NH₃⁺), then an acid addition salt may be formed with a suitable anion. Examples of suitable inorganic anions include, but are not limited to, those derived from the following inorganic acids hydrochloric acid, nitric acid, nitrous acid, phosphoric acid, sulfuric acid, sulphurous acid, hydrobromic acid, hydroiodic acid, hydrofluoric acid, phosphoric acid and phosphorous acids. Examples of suitable organic anions include, but are not limited to, those derived from the following organic acids: 2-acetyoxybenzoic, acetic, ascorbic, aspartic, benzoic, camphorsulfonic, cinnamic, citric, edetic, ethanedisulfonic, ethanesulfonic, fumaric, glucheptonic, gluconic, glutamic, glycolic, hydroxymaleic, hydroxynaphthalene carboxylic, isethionic, lactic, lactobionic, lauric, maleic, malic, methanesulfonic, mucic, oleic, oxalic, palmitic, pamoic, pantothenic, phenylacetic, phenylsulfonic, propionic, pyruvic, salicylic, stearic, succinic, sulfanilic, tartaric, toluenesulfonic, and valeric. Examples of suitable polymeric organic anions include, but are not limited to, those derived from the following polymeric acids: tannic acid, carboxymethyl cellulose. Such salts include acetate, adipate, aspartate, benzoate, besylate, bicarbonate, carbonate, bisulfate, sulfate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulfonate, naphthylate, 2-napsylate, nicotinate, nitrate, orotate, oxalate, palmitate, pamoate, phosphate, hydrogen phosphate, dihydrogen phosphate, pyroglutamate, saccharate, stearate, succinate, tannate, tartrate, tosylate, trifluoroacetate and xinofoate salts.

For example, if the compound is anionic, or has a functional group which may be anionic (e.g. -COOH may be -COO⁻), then a base salt may be formed with a suitable cation. Examples of suitable inorganic cations include, but are not limited to, metal cations, such as an alkali or alkaline earth metal cation, ammonium and substituted ammonium cations, as well as amines. Examples of suitable metal cations include sodium (Na⁺) potassium (K⁺), magnesium (Mg²⁺), calcium (Ca²⁺), zinc (Zn²⁺), and aluminum (Al³⁺). Examples of suitable organic cations include, but are not limited to, ammonium ion (i.e. NH₄⁺) and substituted ammonium ions (e.g. NH₃R⁺, NH₂R₂⁺, NHR₃⁺, NR₄⁺). Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is N(CH₃)₄⁺. Examples of suitable amines include arginine, N,N'-dibenzylethylenediamine, chloroprocaine, choline, diethylamine, diethanolamine, dicyclohexylamine, ethylenediamine, glycine, lysine, N-methylglucamine, olamine, 2-amino-2-hydroxymethyl-propane-1,3-diol, and procaine. For a discussion of useful acid addition and base salts, see S. M. Berge et al., J. Pharm. Sci. (1977) 66:1-19; see also Stahl and Wermuth, Handbook of Pharmaceutical Salts: Properties, Selection, and Use (2011)

Salts such as pharmaceutically acceptable salts may be prepared using various methods. For example, one may react a compound of Formula 1 with an appropriate acid or base to give the desired salt. One may also react a precursor of the compound of Formula 1 with an acid or base to remove an acid- or base-labile protecting group or to open a lactone or lactam group of the precursor. Additionally, one may convert a salt of the compound of Formula 1 to another salt through treatment with an appropriate acid or base or through contact with an ion exchange resin. Following reaction, one may then isolate the salt by filtration if it precipitates from solution, or by evaporation to recover the salt. The degree of ionization of the salt may vary from completely ionized to almost non-ionized.

### INDUSTRIAL APPLICATION

The compositions of the invention find application in industry as saleable products in the form of sunscreens and other formulations. In particular, the invention helps to meet the needs of the industry by reducing the number of active ingredients as well as their proportions in the final products. For example, the Federal Institute for Risk Assessment (BfR) Institute for risk assessment in Germany published a report entitled "UV-Filters in Sun Protection Products - Opinion of the Federal Institute for Risk Assessment, 6th August 2003" which mentioned the following:
*'Recommendation*
*BfR recommends that UV filters and ingredients should be combined in the formulations in such a way that the number of filters as well as the relative amount of filters used for the protection claimed is kept as low as possible. The permitted maximum concentrations for the individual UVfilters must not be exceeded. Furthermore, the added UVfilters should contribute to the sun protection factor of the finished product. The health safety and skin tolerance of the finished products must be guaranteed.'*

Thus the invention possesses the required industrial application/utility.

Further particular and preferred aspects are set out in the accompanying independent and dependent claims. Features of the dependent claims may be combined with features of the independent claims as appropriate, and in combinations other than those explicitly set out in the claims.

Where an apparatus feature is described as being operable to provide a function, it will be appreciated that this includes an apparatus feature which provides that function or which is adapted or configured to provide that function.

### BRIEF DESCRIPTION OF THE DRAWINGS

Embodiments of the present invention will now be described further, with reference to the accompanying drawings, in which:
Figure 1 shows a bar chart.
Figure 2 shows a bar chart.
Figure 3 shows a bar chart.
Figure 4 shows a bar chart.
Figure 5 shows a bar chart.
Figure 6 shows a bar chart.
Figure 7 shows a bar chart.
Figure 8 shows seven bar charts.
Figure 9 shows a bar chart.
Figure 10 shows a bar chart.
Figure 11 shows a bar chart.
Figure 12 shows five bar charts.
Figure 13 shows five bar charts.
Figure 14 shows a graph.
Figure 15 shows a bar chart.
Figure 16 shows a bar chart and five photographs.
Figure 17 shows a bar chart.
Figure 18 shows a graph.

### DESCRIPTION OF THE EMBODIMENTS

Although illustrative embodiments of the invention have been disclosed in detail herein, with reference to the accompanying drawings, it is understood that the invention is not limited to the precise embodiment and that various changes and modifications can be effected therein by one skilled in the art without departing from the scope of the invention as defined by the appended claims and their equivalents.

### EXAMPLES

The following examples are merely illustrative and do not limit the invention. Amounts of individual compounds are expressed as weight percentages.

### Example 1A :

In a specific embodiment, the invention has the following formulation in the form of cream:
Ingredients w/w
palythine 9%
APIFIL 12%
LABRAFAC 15.5%
Olive oil 7%
EDTA 0.05%
Sodium hydroxide 0.4%
Sorbic acid 1 %
Guava and coconut 0.3%
H20 remaining %

### Example 1B :

In a specific embodiment, the invention has the following formulation in the form of cream:
Ingredients w/w
palythine 9%
APIFIL 12%
LABRAFAC 15.5%
Olive oil 7%
Tocopheryl acetate 0.5%
Hydroxyethyl cellulose 0.2%
EDTA 0.05%
Sodium hydroxide 0.4%
Sorbic acid 1 %
Guava and coconut 0.3%
H20 remaining %

### Example 2A:

In a specific embodiment, the invention has the following formulation in the form of cream:
Ingredients w/w
palythine 0.3%
APIFIL 12%
LABRAFAC 15.5%
Olive oil 7%
EDTA 0.05%
Sodium hydroxide 0.4%
Sorbic acid 1 %
Lavender 0.3%
H20 remaining %

### Example 2B:

In a specific embodiment, the invention has the following formulation in the form of cream:
Ingredients w/w
palythine 0.3%
APIFIL 12%
LABRAFAC 15.5%
Olive oil 7%
Tocopheryl acetate 0.5%
Hydroxyethyl cellulose 0.2%
EDTA 0.05%
Sodium hydroxide 0.4%
Sorbic acid 1 %
Lavender 0.3%
H20 remaining %

### Example 3A:

In a specific embodiment, the invention has the following formulation form of cream:
Ingredients w/w
palythine 4.7%
APIFIL 10%
LABRAFAC 13.5%
Olive oil 5%
EDTA 0.05%
Sodium hydroxide 0.4%
Sorbic acid 1 %
Orange 0.3%
H20 remaining %

### Example 3B:

In a specific embodiment, the invention has the following formulation form of cream:
Ingredients w/w
palythine 4.7%
APIFIL 10%
LABRAFAC 13.5%
Olive oil 5%
Tocopheryl acetate 0.5%
Hydroxyethyl cellulose 0.2%
EDTA 0.05%
Sodium hydroxide 0.4%
Sorbic acid 1 %
Orange 0.3%
H20 remaining %

### Example 4: Preparing the Compositions

Compositions according to examples 1A to 3B are prepared by the process described below (adapted from WO2013/014584 in case any further guidance is needed):
Phases 1 and 3, indicated in the Table below, are dissolved together at 65°C using a stirrer.

| Phase | Component | Base/Active |
|---|---|---|
| Phase 1 | emulsifier | Apifil 13% |
| | emollient | Labrafac 18.5% |
| | carrier | olive oil 8% |
| | antioxidant (when present) | tocopherol acetate 0.5% |
| | Active (palythine) 0.3 to 9% | palythine 5% |
| Phase 2 | water | water 53.22% |
| | gelling agent (when present) | hydroxyethyl cellulose 0.2% |
| | preservative/stabiliser | EDTA 0.05% |
| | preservative | sorbic acid 1% |
| Phase 3 | emollient | Labrafac 1% |
| | preservative/alkali | sodium hydroxide 0.4% |
| Phase 4 | Fragrance (when present) | Fragrance 0.3% |

Phase 2 is dissolved separately at the same temperature, always under continuous stirring. Upon reaching the same temperature, phase 2 is added to phases 1 +3 and the whole is mixed for some minutes at 65°C.
To obtain a product having homogeneous consistency, it is mixed at room temperature and only at the end, then phase 4 is added.

The same process is followed for compositions comprising higher amounts of palythine such as from 0.3% to 25% by weight of the composition of palythine, adjusting the water percentage accordingly.

### Example 5A: Skin-Care Composition

An oil-in-water skin-care composition in accordance with this invention is produced as follows:
Phase A (Oil Phase)
   14% PEG-8 beeswax
   19% caprylic/capric triglyceride
   7% olive oil
   1% tocopherol acetate
   0.4% sodium hydroxide
   9% palythine
Phase B (Water Phase)
   0.5% EDTA
   0.2% hydroxyethyl cellulose
   and deionized water making up 100%

Phase A and Phase B are heated separately at 650C under continuous stirring. Phase B is then added to phase A under vigorous stirring at 650C for 5 minutes, followed by cooling to room temperature still under continuous stirring, until a homologous consistency is reached.

### Example 5B: Skin-Care Composition

An oil-in-water skin-care composition in accordance with this invention is produced as follows:
Phase A (Oil Phase)
   14% PEG-8 beeswax
   19% caprylic/capric triglyceride
   7% olive oil
   1% tocopherol acetate
   0.4% sodium hydroxide
   25% palythine
Phase B (Water Phase)
   0.5% EDTA
   0.2% hydroxyethyl cellulose
   and deionized water making up 100%

Phase A and Phase B are heated separately at 650C under continuous stirring. Phase B is then added to phase A under vigorous stirring at 650C for 5 minutes, followed by cooling to room temperature still under continuous stirring, until a homologous consistency is reached.

### Example 6: Anti-Ageing Skin-Care Composition

The following components are used to make an anti-ageing skin-care composition of the present invention:
Water, caprylic/capric triglyceride, octyldodecanol, microcrystalline cellulose, xanthan gum, glycerin, isoamyl laurate, polyglyceryl-3-methylglucose distearate, methylheptyl isostearate, prunus amygdalus dulcis oil, olea europaea leaf extract, stearic acid, glyceryl stearate, Sambucus nigra flower extract, tocopheryl acetate, retinyl palmitate, ascorbic acid, perfume, citral, limonene, jasminum officinale oil, maltodextrin, sodium phytate, sodium benzoate, potassium sorbate, sodium hydroxide, and palythine.

Suitably palythine is present at 0.3-25% by weight.

### Example 7: Isolation of palythine

palythine was isolated using the method described in the following reference, (which is incorporated herein by reference specifically for the method of preparation of palythine):
Isami Tsujino, Kazuo Yabe, Isao Sekikawa, Nobuyuki Hamanaka (1978) Isolation and structure of a mycosporine from the red alga Chondrus yendoi. Tetrahedron Letters (Issue 16) Pages 1401-1402.

Approximately 2.5 mg of palythine can be extracted from 10 g of dried *Chondrus yendoi.* This is an edible Japanese seaweed also known as Kuroha- Ginnanso and is available commercially e.g. Hokuyo-syokusan Co Ltd, Hokkaido, Japan (http://www.hoshitako.co.jp/index.html).

The seaweed is cut into small (0.5 cm²) pieces and extracted overnight at room temperature with gentle shaking in 0.5 L of 80 % aqueous ethanol. The extract is passed through a course filter to remove any seaweed debris and dried under pressure at below 40 °C. The residue is dissolved in approximately 10 ml water and slowly applied to a 50 cm x 1 cm column containing 10 g activated carbon. The carbon is washed with water until no chloride ions are detected in the flow-through by measuring fractions at 320-330 nm. palythine is eluted using 50 % aqueous methanol. High UV 320 nm absorbing fractions are then pooled;dried under pressure at below 40 °C and the residue taken up in absolute methanol and the concentration process repeated until all the water is removed to yield white crystals with a melting point of 155-156 °C.

### Example 8: Beneficial Characteristics

### In-vitro testing:

The following *in vitro* tests using the immortalized human skin keratinocyte cell line HaCaT human have been performed using purified palythine isolated from *C*. *yendoi*: (It is believed that the same tests may be applied to dermal fibroblasts is desired):

### DNA photodamage

This measurement is based on the comet assay (single cell gel electrophoresis) which detects damage to nuclear DNA that result from nucleotide excision repair (NER) and nucleotide photooxidation. Using the lesion specific enzymes T₄N₅ that recognizes CPD and hOGG1 that recognizes 8-oxoGua, palythine was tested by comparing pure palythine compound with naturally occurring amphoteric MAA mixtures to that of industry accepted sun-filter controls. We have shown that palythine significantly reduces DNA damage caused by both UVA and UVB exposure. More specifically, Using the lesion specific enzymes T₄N₅ which recognizes CPD and hOGG1 which recognizes 8-oxoGua, we have shown that palythine significantly reduces DNA damage caused by UVA exposure in comparison to naturally occurring "non-acidic" MAA mixtures and industry accepted sun-filter controls.

### Reactive Oxygen species (ROS)

Intracellular ROS generation was measured using the detection agent 5-(and-6)-carboxy-2',7'- dichlorodihydrofluorescein diacetate (carboxy-H2DCFDA). Again, palythine was shown to significantly reduce total intracellular ROS generated after exposure to both UVA and UVB exposure by comparing pure palythine compound with naturally occurring amphoteric MAA mixtures to that of industry accepted sun-filter controls. More specifically, total intracellular ROS generation was studied using 5-(and-6)-carboxy-2',7'-dichlorodihydrofluorescein diacetate (carboxy-H2DCFDA). Again, palythine was shown to significantly reduce total intracellular ROS generated after exposure to UVA exposure in comparison to naturally occurring "non-acidic" MAA mixtures and industry accepted sun-filter controls.

### Palythine & Salmon roe extract MAA - Methods and Results

### Methods

### Cell Culture

HaCaT cells (ATCC, USA) were maintained in DMEM (Invitrogen, Paisley, UK) supplemented with 10% FCS (Sigma-Aldrich, UK) and 1% Penicillin/streptomycin (Invitrogen)

### Powdered Sunscreens Treatments in vitro

Powdered sunscreens were dissolved in PBS (Sigma-Aldrich, UK) at a concentration in which they can dissolve fully (0-0.3%). They were then applied (200ul/well) to wells of a 24 well plate containing a washed monolayer of cells. Cells were then irradiated and the sunscreen removed and washed post treatment.

### Irradiation of cells

Irradiation was carried out on cells in monolayers at a distance of 3 cm (Solar Simulator) in cold PBS or PBS + sunscreen. The plate was always placed on a cooling platform at 4°C to avoid overheating of the cells. Unirradiated samples were maintained in PBS for the same time and temperature as irradiated samples.

### Single-cell gel electrophorese assay (Comet Assay)

DNA damage was measured using the comet assay, using lesion specific enzymes to determine the type of damaged produced.

Glass slides are pre-coated with 1% agarose and allowed to dry. Cells were treated with UV +/- sunscreens. Positive controls were treated with ice cold 0.03% Hydrogen Peroxide for 5 minutes, negative controls were kept in the same conditions as the treatments without UV exposure.

After treatment cells were scraped gently with a cell scraper in EDTA solution to help dissociate cells and stop clumping. The cells (40ul) were then added to low melting point (LMP) agarose (160ul) and gently mixed. The final concentration of cells in the LMP agarose was 10,000 cells/100ul of agarose. Then 100ul of the agarose-cell solution was pipetted onto the pre-coated slides and a cover slip was placed onto the gels and left to set for 5-10 minutes then removed. Two replicates were placed on each slide. All steps were carried out on a cooled platform at 4°C. Slides were then places into lysis buffer (2.5M NaCl, 100mM EDTA, 10mM TRIS, 1% sodium sarcosinate, 1% Triton x-100, 10% DMSO) overnight at 4°C in the dark.

Slides were then washed in ice-cold ddH₂O (2x5 mins) in the dark and once in ice cold enzyme reaction buffer (0.4mM HEPES, imMKCl, 0.005mM EDTA, 0.02mg/ml BSA) for 5 minutes. After this step different lesions were detected using lesion specific enzymes.

Two lesions were measured, 8-oxoguanine caused by oxidatively induced DNA damage was measure using the human 8-oxoguanine DNA glycosylase (hOGGi) enzyme. Cylcobutane pyrimidine dimers were measured using the T4 endonuclease V (T₄endoV) enzyme.
After washing, 50ul of enzyme diluted in reaction buffer (hOGG1- 3.2U/ml, T₄EndoV - 0.1U/ml) or reaction buffer alone were added to each gel, a cover slip placed on top and left in a humidity chamber in the dark at 37°C for 45 minutes.

After enzyme treatment coverslips were removed and transferred into ice-cold electrophoresis buffer (10mM NaOH, 200mM EDTA, pH13 in ddH₂O) and incubated at 4°C in the dark for 20 minutes. Electrophoresis was then carried out in fresh electrophoresis buffer for 25 minutes at 25V (1V/cm) 300mA, at 4°C in the dark. Slides were then washed in neutralisation buffer (0.4M TRIS, pH7.5) for 2x 10 minutes, and then in ddH₂O for 2x 5 minutes and slides were allowed to dry overnight.

The DNA embedded in the slides was stained using Propidium Iodide solution (2.5ug/ml in PBS), 50ul of the solution was added to each gel and cover slip placed over. The slides were then analysed on a Zeiss Axiophot epifluorescence microscope (Carl Zeiss, Germany), with a green excitation filter (536nm) detected with a red emission at 617nm. Pictures covering the whole gel were taken, avoiding the outer edges, using a Nikon camera at a magnification of 10x. The images were then analysed using Comet Score Pro software (Tritek Corp, Summerduck, VA), typically scoring 150 individual comets per condition. The percentage of DNA in the tail compared to the total DNA present in the whole comet was the parameter used to determine the damage of each condition.

### 2.2.7 Reactive Oxygen Species ROS Detection by Fluorescent Activated Cell Sorting (FACS) Analysis

Detection of reactive oxygen species was performed by FACS analysis using 5-(and 6-)-carboxy-2,',7'-dichlorodihydrofluorescein diacetate (Carboy-H2-DCFDA) (Invitrogen, Paisley, UK). Carboy-H2-DCFDA is a non-fluorescent dye that is activated into a fluorescent dye by ROS cleaving acetate groups from the molecule.

Cells were grown in a 12 well plate washed twice in PBS and irradiated +/- sunscreens. Control cells were unirradiated and positive controls were treated with 0.03% hydrogen peroxide for 5 minutes. Cells were washed again twice in PBS and then 1ML of Carboy-H2-DCFDA diluted in PBS to a concentration of 5uM was added to each well and were incubated for 2 minutes in a cell culture incubator in the dark (95% air, 5% CO₂, 37°C, humidified). Each well was then washed twice with PBS to remove any excess dye.

Cells were then trypsinised, centrifuged at 400g for 4 minutes at 4°C and resuspended in 0.5ml of PBS + 0.1%w/v BSA and transferred to FACS tubes. Samples were then analysed with a Becton Dickson CANTO 3. The viable proportion of the cell population was quantified by the addition of 2.5ug/ml of propidium iodide immediately before the analysis.

### Results

### palythine

**Comet Assay - Single strand breaks / Alkali labile sites**

| | **palythine conc. condition** | **Mean % DNA Damage** |
|---|---|---|
| **No Enzyme** | 0 | 22.881 |
| | 0.1 | 14.263 |
| | 0.3 | 9.914 |

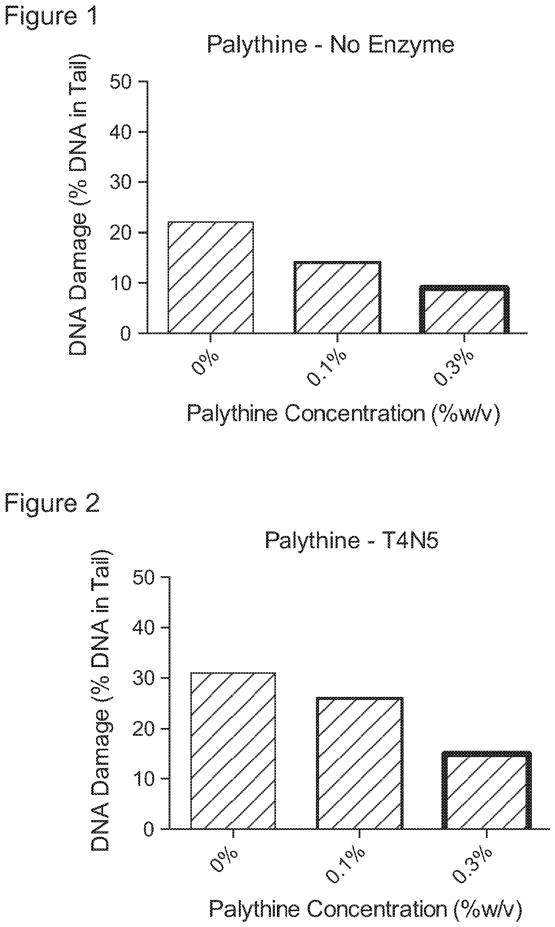

HaCaT keratinocytes were irradiated with 10J/cm² UVA with no 0% (PBS alone), 0.1% and 0.3% palythine in PBS. The mean percentage of tail DNA (Percentage damage) was plotted against sunscreen concentration (n=1). See Figure 1.

**Comet Assay - Cyclobutane Pyrimidine dimers (CPDs)**

| | **palythine conc. condition** | **Mean % DNA Damage** |
|---|---|---|
| **T4N5** | 0 | 31 |
| | 0.1 | 26.328 |
| | 0.3 | 15.345 |

HaCaT keratinocytes were irradiated with 10J/cm² UVA with no 0% (PBS alone), 0.1% and 0.3% palythine and then treated with T4endoV enzyme to introduce strand breaks at CPD lesions. See Figure 2.

**FACS Analysis - Reactive Oxygen Species**

| **Condition** | **Median corrected Average** |
|---|---|
| **Negative** | 34.05 |
| **Positive** | 43.3 |
| | |
| **0% palythine** | 38.55 |
| **0.1% palythine** | 36.35 |
| **0.3% palythine** | 33.9 |

See Figure 3.

### Salmon Roe MAAs

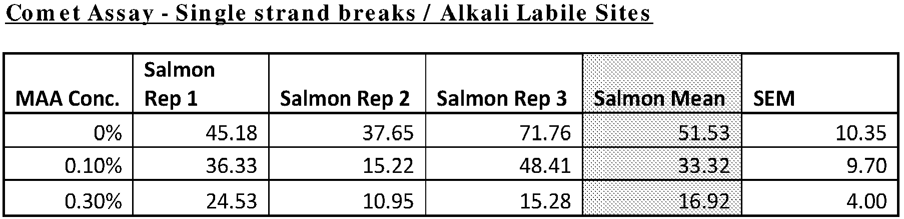

See Figure 4.

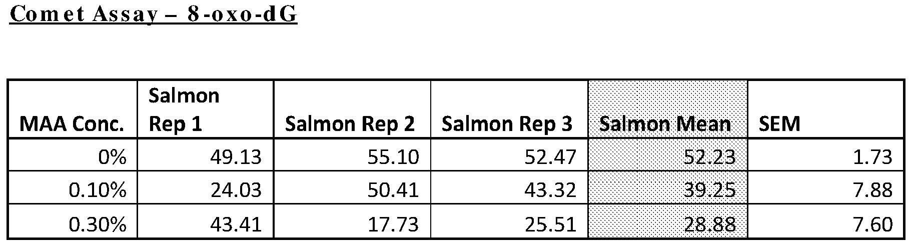

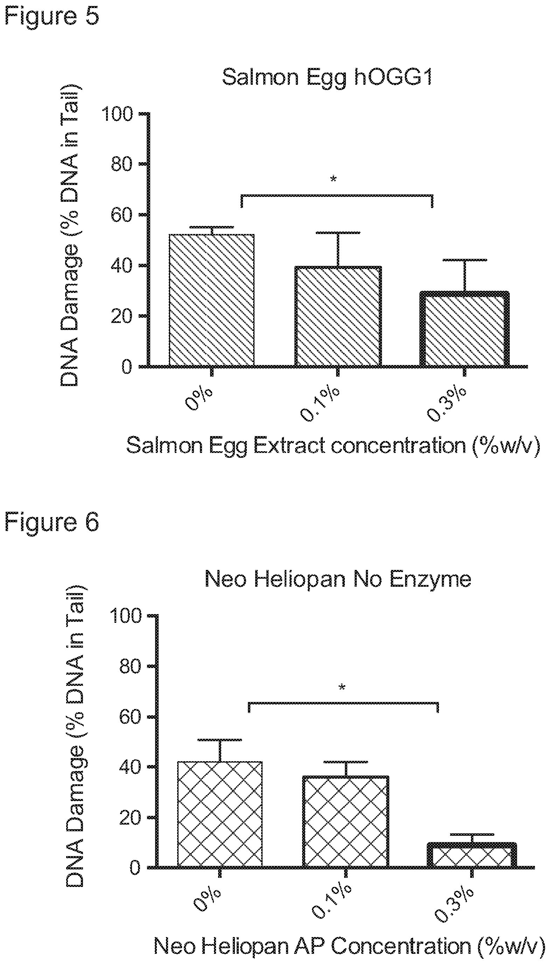

See Figure 5.

### Neo Heliopan AP

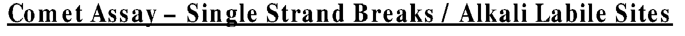

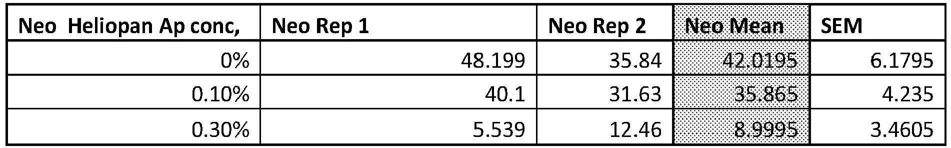

See Figure 6.

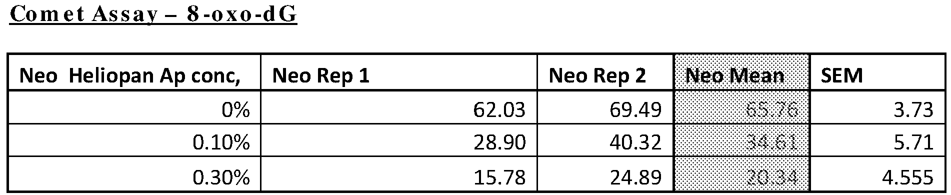

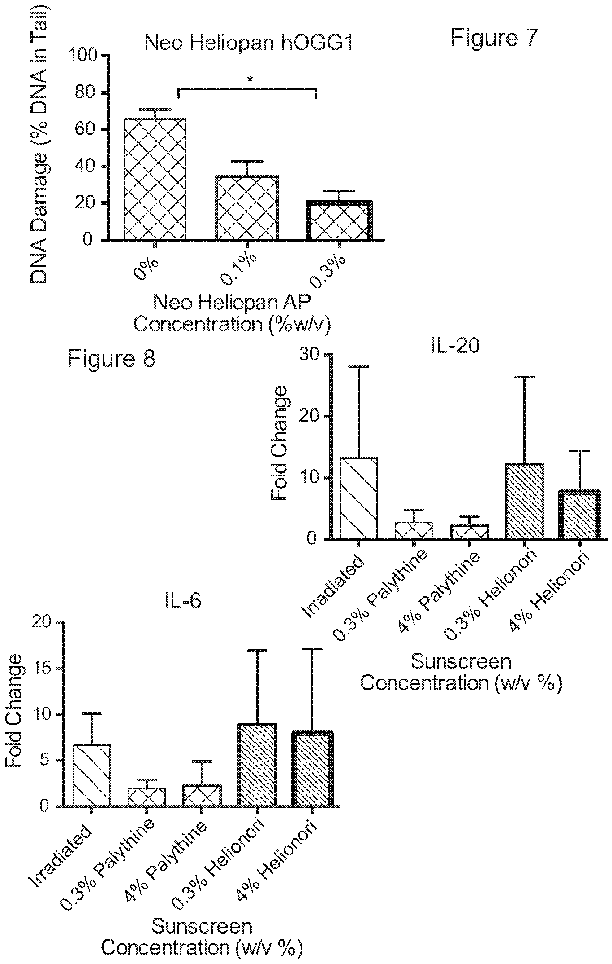

See Figure 7.

### Example 9: Comparative Example

There are 3 commercially available products on the market that use MAAs. None of these is a consumer based product, they are available to purchase in bulk for third party formulations, of which there is only one currently available (dr. brandt™ UV SPF 30 high protection face).

All three bulk products use an extract from the same seaweed *Porphyra umbilicalis*:
Helioguard-365 (Mibelle, Switzerland) uses a mixture of MAAs at 0.1% w/v
Noriguard-nc (Mibelle, Switzerland) uses a mixture of MAAs at 0.05% w/v
Helionori (Gelyma) uses a mixture of MAAs at between 1-4% w/v.

Mibelle product literature lists the mixture as shinorine and porphyra. They state not to be able to detect palythine.

Gelyma product literature lists the mixture as shinorine, porphyra and palythine, but do not state the amount of each.

The most comprehensive study [Helgol Mar Res (2009) 63:231-238] of this seaweed for MAA composition lists the mixture as:
palythine 3% (0.29 mg/g dry weight seaweed)
Shinorine 7% (0.62 mg/g dry weight seaweed)
Porphyra 90% (8.52 mg/g dry weight seaweed)

So to calculate the maximum possible palythine in the prior art composition, we take 4% as the highest concentration of the mixture used by Gelyma: that is 4g mixture/100ml composition which is equivalent to 0.12 % w/v palythine.

From our data, this would result in 76% DNA damage.
This figure of 76% damage for the known Gelyma composition compares to only 48% damage at 0.3% palythine, which is what we teach as the lowest effective amount of palythine as active ingredient in compositions of the invention.
Thus the nearest prior art composition at its highest possible concentration allows 76% DNA damage. By contrast, the present invention teaches a minimum level of palythine of 0.3% - this minimum level provides almost 60% improved (improving from 48% to 76% damage = 28 percentage points improvement i.e. 58.33% better) protection from DNA damage.

In fact, Gelyma actually only used 2% w/v in their DNA damage test, so at a maximum of 0.06% w/v palythine in the Gelyma composition we estimate 86% DNA damage. In this case, the composition of the invention having the minimum effective amount of palythine taught herein of 0.3% provides enormously improved protection from DNA damage (i.e. from 48% damage for the composition of the invention having 0.3% palythine compared to 86% damage for the known Gelyma composition is a rise of 38 percentage points; therefore the known Gelyma composition allows nearly four-fifths or 180% more DNA damage than the beneficial compositions of the invention.)

### Example 10 : MAA Gene expression

The gene expression analysis was performed on HaCat cells exposed as previously described. Following irradiation growth media was replaced RNA extraction was carried out 12 hours later. RNA extraction was carried out using the mirVana miRNA Isolation kit (Life Technologies, Paisley, UK) according to the manufacturers protocol for total RNA extraction. RNA quality was tested using RNA 6000 Nano Kit according to the manufacturers instruction.

Total RNA was then converted to cDNA using the High Capacity cDNA Reverse Transcription Kit (Applied Biosystems, Paisley, UK) according to manufacturers instructions. qPCR was performed using TaqMan Gene Expression Assays (Applied Biosystems) according to the manufacturers protocol with the following TaqMan FAM labelled DNA probes IL-20, IL-8, IL-6, TFRC, HMOX-1, MMP-3, MMP-12. VIC labelled GAPDH was used as the housekeeping gene. For each gene the fold change was measured using the ΔΔCT method.

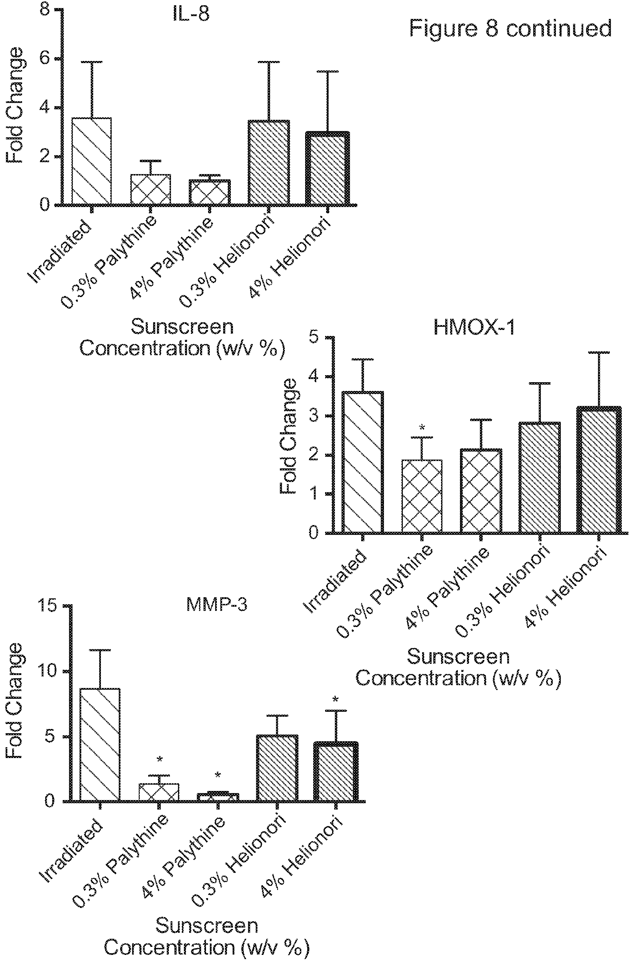

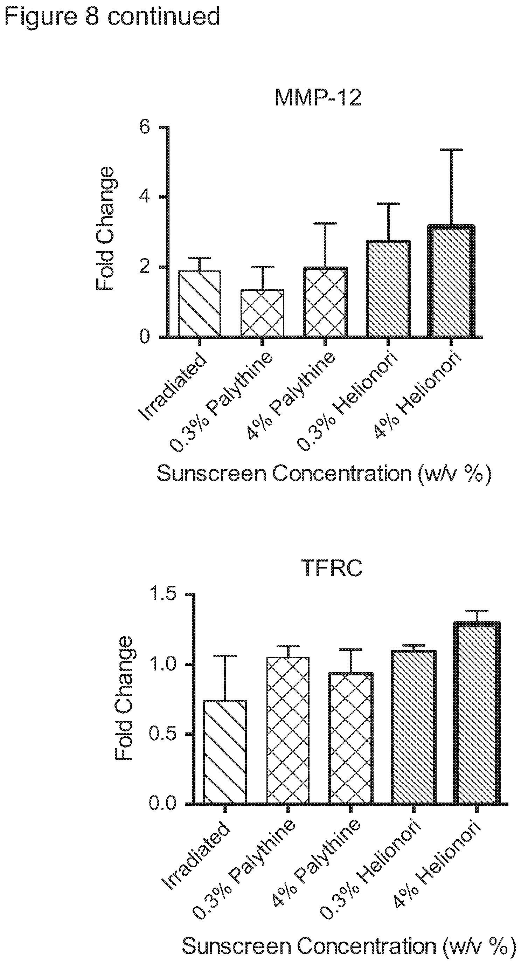

Results are shown in the seven bar charts presented in Figure 8.

Graphs are representative of the average fold change for at least 4 experiments ± SD.
4% 'Helionori' mixture = 0.12% palythine.

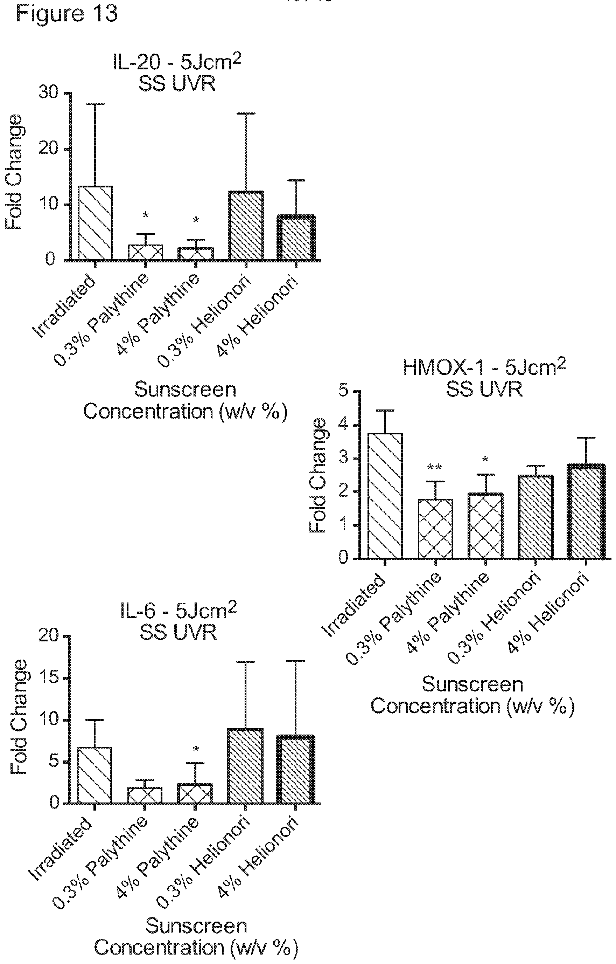

The MMP-12 and TFRC data seem similar across treatments - for these genes the palythine and helionori had no effect on reducing gene expression compared to the control. It is not possible to predict which genes will be responsive to this UVR exposure. The inventors tried several genes with varying results as shown. Clearly if there is no UVR-induced change of expression (Figure 8 - MMP-12 and TFRC data), those genes are not useful in demonstrating protection. Therefore when testing/ demonstrating protection, genes not responsive to UVR alone are not useful as they do not show a UVR-induced effect. For this reason, in generating additional data (Figure 13 - see example 13 below), the inventors did not include genes that had not previously responded to UVR alone.

Regarding HMOX-1, 0.3 % palythine significantly reduced expression of the gene compared to 4 % palythine and both concentrations of helionori compared to the control.
The asterisks signify statistical significance.

### Example 11: DNA Damage

### Comet Assay Results

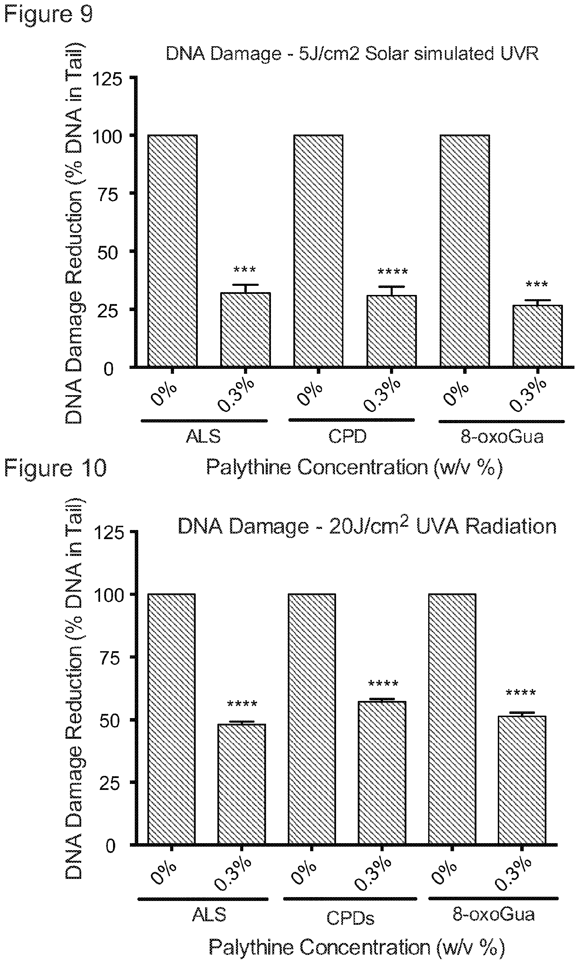

HaCaT keratinocytes were irradiated with 5J/cm² solar simulated UVR with 0% (PBS alone), and 0.3% of palythine. The data are shown in figure 9, which illustrates protection by palythine against solar simulated UVR induced DNA damage measured by comet assay.

The percentage change in % DNA in the tail in protected cells compared to unprotected cells was plotted ± SD. (n=3, ≥50 comets scored per condition per experiment). The significance of change in DNA damage was analysed by t-test. It was found that there was significant protection when using 0.3% of palythine compared to unprotected cells. Significance is expressed by *.

HaCaT keratinocytes were irradiated with 20J/cm² UVA with 0% (PBS alone), and 0.3% of palythine. The data are shown in figure 10, which illustrates protection by palythine against UVA induced DNA damage measured by comet assay.

The percentage change in % DNA in the tail in protected cells compared to unprotected cells was plotted ± SD. (n=3, ≥50 comets scored per condition per experiment). The significance of change in DNA damage was analysed by t-test. It was found that there was significant protection when using 0.3% of palythine compared to unprotected cells. Significance is expressed by *.

### IHC-IF Results

HaCat keratinocytes were irradiated with 20J/cm² of solar simulated UVR radiation with 0% (PBS alone), 0.3%, 4% or 10% of palythine. The relative formation of the CPD DNA lesion was measured by IHC-IF and the relative fluorescent intensity plotted. The data in figure 11 show protection by palythine against solar simulated UVR induced CPD formation by IHC-IF.

### DNA Damage

The radiation procedure was carried out as described above. Cells were washed, then fixed in 2 % (v/v) paraformaldehyde with 0.5 % (v/v) Triton X-100 in PBS for 30 mins at 4 °C. DNA was then denatured by incubation in 2 M HCl for 10 mins at 37 °C. Nonspecific sites were blocked using blocking buffer of 20 % (v/v) goat serum and 0.1 % (v/v) triton x-100 in PBS for 30 mins at room temperature. The primary antibody used was Anti-CPD (Clone TDM-2) (Cosmobio, Tokyo, Japan) at 1:1000 in blocking buffer for 1 hr at room temperature. Alexafluor 488 was diluted in blocking buffer (1:200 dilution) and incubated for 1 hr at room temperature and finally DAPI was added for 10 mins. Washing was carried out (3x5 mins) between each step.

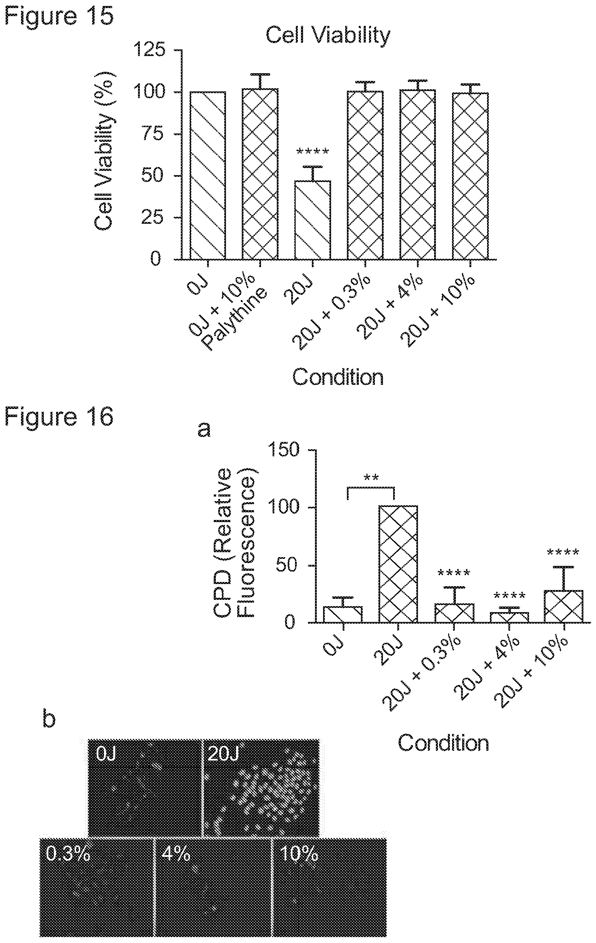

Referring to figure 16, HaCaT keratinocytes were significantly protected from SS UVR and UVA induced CPD, 8-oxoGua and ALS DNA damage by palythine at a range of concentrations, (a, b) HaCaT keratinocytes were untreated, exposed to UVR (20J/cm² SSR) with 0%, 0.3, 4 or 10% w/v palythine. CPDs were measured immediately post exposure using IHC-IF. Columns represent mean ± SD (n=3). Cells irradiated without palythine showed a significant increase in CPD production (p=0.0029, paired t test) compared to the unirradiated control. Palythine at 0.3-10%w/v showed a significant reduction in CPD production compared to irradiated control (p<0.0001, one-way ANOVA with Dunnetts multiple comparisons test). (b) Typical fluorescent images for each condition.

### Example 12: Gene expression

Cells irradiated with 5J/cm² SSR with 0%, 0.3%, 4% and 10% palythine. RNA was extracted, cDNA synthesised, and gene expression analysed by qCR. Figure 12 presents data for the various treatments (including 10% data presented for HMOX-1, IL-6, IL-8) which demonstrate protection by palythine against solar simulated UVR induced gene expression.
The mean fold change ± SD was analysed with n≥3. The difference compared to 0% irradiated sample was analysed using ANOVA. Significance is expressed by *.

### Example 13: Comparative Study

Compositions of the invention having palythine as the sole active ingredient were compared to compositions comprising the prior art *Helionori* (soluble extract of red algae which contains MAAs - discussed above).

Cells irradiated with 5J/cm² SSR with 0%, 0.3% & 4% palythine or *Helionori.* RNA was extracted, cDNA synthesised, and gene expression analysed by qPCR.
Data presented in figure 13 show the protection offered by palythine according to the invention compared with protection offered by prior art composition *Helionori,* as assessed by UVR induced gene expression.

The mean fold change ± SD was analysed with n≥3. The difference compared to 0% irradiated sample was analysed using ANOVA. Significance is expressed by *.

### Example 14: Photostability and protection offered by mycosporine-like amino acid palythine as a sunscreen.

### Background:

Although sunscreens have provided many benefits to human health in reducing sunburn and skin cancer incidence, it has been found that they are potentially damaging to the environment, particularly to marine species and coastal waters. This has led to a need to find biocompatible sun screening products.
Many marine microorganisms, such as cyanobacteria, contain natural, inbuilt, UVR protection in the form of mycosporine-like amino acids (MAAs), which are produced to protect organisms in environments of high UVR exposure. These compounds have been found in larger fish, particularly in the ocular and epidermal tissues, and eggs. The inventors hypothesised that these MAAs are accumulated in the food chain and provide protection against UVR to higher species. As well as absorbing UVR, these compounds have been shown to have antioxidant properties.

### Aims

To extract the MAA palythine from the red algae *Palmaria decipiens* and test its purity using HPLC and LC-MS
To determine palythine's absorbance profile and photostability.
To deomonstrate palythine as a potential sunscreen filter and antioxidant investigating different endpoints: DNA damage, reactive oxygen species, gene expression & SPF.

### Methods

palythine was extracted from the red algae *Palmaria decipiens* using a methanol extraction (Confirmed by HPLC & LC-MS). The absorbance profile was determined and then the extract was exposed to increasing doses of solar simulated radiation (SSR) from a solar simulator (conforming to COLIPA standards). The absorbance profile was measured to determine photostability.
Compositions according to the invention (in this example solutions of palythine) were prepared at different palythine concentrations (0.3% to 10%), as well as controls comprising 0% palythine, and HaCat keratinocytes were irradiated with SSR or UVA radiation through the solutions, which were placed on top of the cells.

DNA damage was then measured using the comet assay and IHC-IF looking at the specific DNA lesions of cyclobutane pyrimidine dimers (CPD) and 8-oxoGua. A percentage reduction in general and specific DNA damage was then determined by comparing cells irradiated with and without sunscreen compositions. Antioxidant ability was detected using H2DCFDA, which fluoresces when reacting with ROS with the addition of palythine post exposure.
SSR induced gene expression covering a range of different markers (inflammation, photoageing, oxidative damage) was assessed using qPCR. The ability of palythine to reduce this gene expression was then assessed.

### Results

The MAA palythine was found to be extremely photostable, even after 60 standard erythema doses (SED), absorbing in the UVB and UVA ranges (290-350nm).
The results of the comet assay and IHC-IF investigating DNA damage found that palythine at 0.3% or greater provided significant protection for the DNA lesions tested (general damage, CPD and 8oxoGua) at all concentrations tested for UVA and SSR induced damage (p=<0.05).
SSR induced differential gene expression for all markers investigated was found to be significantly reduced with the presence of palythine at 0.3% or greater at all concentrations (p=<0.05)
palythine was also found to possess anti-oxidant properties with a significant reduction in ROS production due to SSR irradiation (p₌<0.05).

### Conclusions

In conclusion, the MAA palythine appears to be extremely effective photostable active ingredient for use in sunscreens e.g. to be used as a biocompatible sunscreen filter by significantly reducing SSR and UVA-induced DNA damage, oxidative damage, gene expression and ameliorating or reducing damage to marine ecosystems.

### Overview of Examples 1-14

It is clearly demonstrated that compositions of the invention provide statistically significant protection as sunscreens. In particular we show that 0.3% (and greater) palythine is effective, whereas <0.3% palythine such as in certain prior art compositions does not work. Direct comparative data are provided in the form of gene expression studies, shown by the fact that 4% *Helionori*, (which contains ∼0.06% palythine), shows no protection whereas compositions of the invention comprising 0.3% palythine show significant protection. This is indicated by a * on the graphs and is defined as a significant decrease in expression compared to the irradiated control. This is especially clear in figure 13.

We also show a range of other higher palythine concentrations (within the ranges of the invention) working. This includes gene expression studies carried out at 0.3%, and various concentrations upwards including 10% palythine, which show a significant decrease in the expression of the genes compared to the irradiated controls. DNA damage also shows a decrease across the range. (especially clear in figures 9 to 12). Assessment of DNA damage and various of the gene expression markers show protective benefit at various exemplary palythine concentrations according to the invention. Thus we present representative data across the range of palythine concentrations according to the invention.

In more detail, the gene expression data (especially figure 12) show:
- Markers of Inflammation:
   IL-20 - Inflammatory cytokine (also found to be upregulated in psoriatic skin conditions)
   IL-6 - pro inflammatory cytokine
   IL-8 - chemokine (linked to psoriasis and oxidative stress)
- Marker of Ageing:
   MMP-3 - Involved in the breakdown of extracellular matrix and tissue remodelling by degrading collagen (the major structural protein in skin)
- Markers of Oxidative Stress:
   HMOX-i - Upregulated in response to oxidative stress
- Markers of DNA damage:
   Comet assay (figures 9 and 10) - Method of measuring DNA damage. Results show a significant reduction in specific DNA lesions (CPD, 8oxoGua) when cells are irradiated in the presence of palythine compared to irradiated control sample.

Immunohistochemistry immunofluorescence (IHC-IF) (Figure 11) - Method of fluorescently staining for specific DNA damage - in this case CPD.
CPD are the major DNA lesion induced by UVR.

8oxoGua are oxidative DNA lesions which are induced by oxidative stress due to UV exposure.

With regard to the comparative data showing advantageous effects compared to the prior art, it is shown via the gene expression studies that irradiated cells show a significant increase the in the above genes. There is a significant reduction in the expression in the genes when tested with palythine compared to the irradiated control (containing no sunscreen) (denoted by *). With the prior art *Helionori*, there is no significant difference between the irradiated control and irradiation with prior art *Helionori.* This suggests that using prior art *Helionori* offers no additional protection compared to using no protection at all, and that by contrast using compositions of the invention comprising palythine offers significant protection.

### Example 15

### Irradiation Procedure for all methods

All assays were performed on HaCat cells grown as a 70-80 % confluent monolayer in multiwell plates. They were were washed three times in PBS and covered with palythine dissolved in PBS at differing concentrations (0, 0.1, 0.3 %, 4 % and 10 % w/v). The cells were then irradiated with either SSR or UVA radiation (5-20 J/cm² or 20 J/cm² respectively). After irradiation the palythine solution was removed and the cells washed a further three times and replaced in media or processed immediately depending on the experimental design.

### Example 15A: Cell Viability

Cell viability was measured 24 hours post irradiation using the neutral red uptake assay. Neutral red solution (4µgml⁻¹ in growth medium) (Sigma, UK) was added to the cells and they were incubated at 37 °C, 5 % CO₂ for 2 hrs. Cells were washed three times in PBS to remove excess neutral red solution and then the destain solution (50 % v/v ethanol, 49 % v/v ddH₂O, 1 % v/v glacial acetic acid) was added. Optical density was measured at 540 nm.

Referring to Fig 15, HaCaT keratinocytes were significantly protected by palythine from UVR induced cell death. HaCaT keratinocytes were untreated, exposed to 20J/cm2 of SSR with PBS alone, 0.3 % (w/v), 4 % (w/v) or 10 % (w/v) of palythine or 10 % (w/v) palythine without exposure to UVR. Cell viability was measured 24 hrs later by the neutral red assay. Columns represent the mean ± SD (n=3). Palythine provided significant protection at all concentrations against cell death, without exposure and cells exposed without palythine had significantly reduced viability (p=<0.0001, One-way ANOVA with Dunnetts multiple comparisons test).

### Example 16: Reactive Oxygen Species Assay

In this example, we demonstrate UV filtering AND antioxidant effect for palythine such as palythine compositions of the invention.
HaCat cells were irradiated as above or for the anti-oxidant studies, cells were also incubated with palythine post irradiation. Cells were then incubated with 10uM carboxy-H2DCFDA (Invitrogen, UK) in PBS for 30 mins in the dark at 37 °C, 5 % CO₂. Cells were washed in PBS, trypsinised for 10 minutes at 37 °C, centrifuged at 1200 rpm for 5 minutes at room temperature and resuspended in PBS and counterstained with DAPI for analysis by FACS. This was carried out with a Becton Dickinson FACSAria II. Cells were gated to only analyse live cells (DAPI negative) and the average mean green intensity per condition was then plotted from at least 10,000 measured events. Analysis was carried out using FlowJo 8.7 (USA).

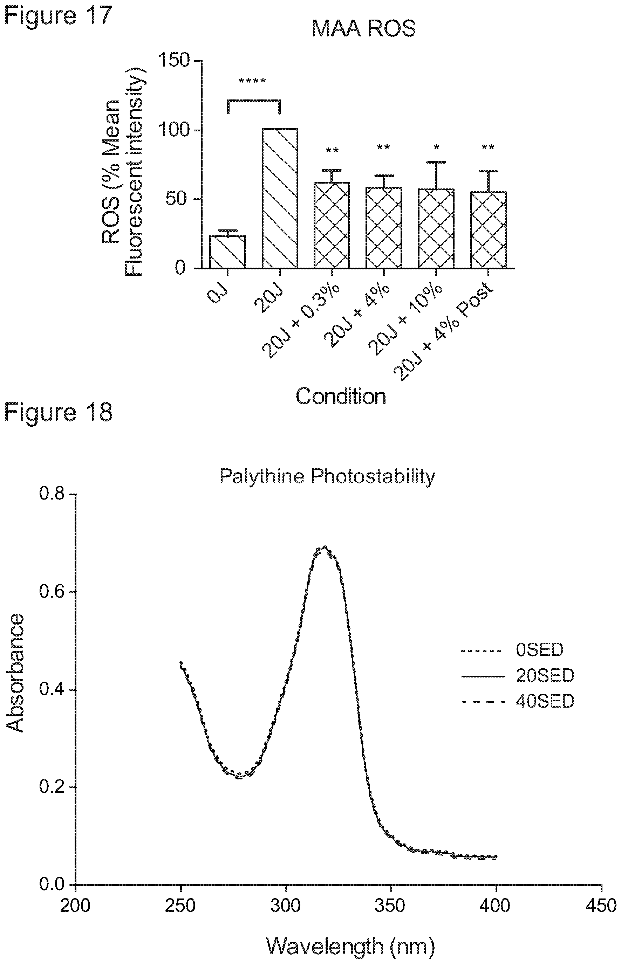

Referring to figure 17, palythine provided significant protection from UVR induced ROS induction and exhibited anti-oxidant properties in HaCaT keratinocytes. HaCaT keratinocytes were untreated, exposed to 20J/cm² of SSR with PBS alone, 0.3%, 4% or 10% palythine or 10% palythine post exposure. ROS levels were measured by FACS to record the mean fluorescent intensity for each condition after cells were treated carboxy-H2DCFDA. Columns represent the mean ± SD (n=5). Cells irradiated without palythine showed a significant increase in ROS (p<0.0001, paired t test) compared to the unirradiated control. Palythine at 0.3-10%w/v showed a significant reduction in ROS production compared to irradiated control when added pre exposure or at 10% post exposure (p<0.0001, one-way ANOVA with Dunnetts multiple comparisons test).

### Example 17: Photostability Data

Palythine was exposed to increasing doses of solar simulated radiation and absorbance measured. The absorbance spectrum of the palythine was measured between 280-400 nm using a Spectra Max 384 Plus (Molecular Devices; California, USA) spectrophotometer.
Referring to figure 18, we show the photostability of palythine after exposure to increasing doses of solar simulated radiation. Palythine (0.0001% w/v palythine extracted from red algae *Chondrus yendoi* in PBS) was exposed to increasing doses of solar simulated radiation and the absorbance was measured to determine photostability. Even after 40SED of exposure (equivalent to around 7 hours of peak UK summer sun at midday) palythine remained extremely photostable with negligible photodegradation.

### Example 18: Moisturising Cream

MoisturIsing FACE Cream Formulations to contain Palythine FOR 30-50SPF SPF

**Formula 1**

| | |
|---|---|
| Aqua | 38.5% |
| PALYTHINE | 20.0% |
| Candelilla/Jojoba/Rice Bran Polyglyceryl-3 Esters | 7.0% |
| Glyceryl Stearate | 4.8% |
| Glycerin | 1.5% |
| Cetearyl Alcohol | 3.2% |
| Sodium Stearoyl Lactylate | 3.4% |
| Caprylic/Capric Triglycerides, | 4.3% |
| Butyrospermum Parkii Butter | 1.8% |
| Vitis Vinifera Seed Oil | 4.2% |
| Prunus Armeniaca Kernel Oil | 2.3% |
| Palmitic/Stearic Triglyceride | 0.8% |
| Cera Alba | 1.3% |
| Amaranthus Caudatus Seed Oil | 0.6% |
| Hippophae Rhamnoides Extract | 1.1% |
| Argania Spinosa Oil | 0.3% |
| Helianthus Annuus Seed Oil | 0.2% |
| Lycium Barbarum Fruit Extract | 0.4% |
| Mimosa Tenuiflora Bark Extract | 0.3% |
| Sodium Benzoate | 1.0% |
| Potassium Sorbate, | 0.4% |
| Tocopherol | 2.0% |
| Rosmarinus Officinalis Leaf Extract, | 0.3% |
| Aroma | 0.3% |

This moisturising cream may be manufactured by Centisia Laboratorio di Fitocosmesi di Bruno Dott.ssa Laura Francesca & C. S.a.s. of Via XXV Aprile, 83, Laveno-Mombello VA, Italy.

### Example 19: Moisturising Cream

MoisturIsing FACE Cream Formulations to contain Palythine FOR 30-50SPF SPF

**Formula 2**

| | |
|---|---|
| Aqua | 45.5% |
| PALYTHINE | 15.0% |
| Caprylic/Capric Triglycerides | 7.4% |
| Glyceryl Stearate Citrate | 7.8% |
| Glycerin | 6.5% |
| Cocos Nucifera Oil | 3.2% |
| Cera Alba | 1.5% |
| Prunus Armeniaca Kernel Oil | 1.2% |
| Palmitic/Stearic Triglyceride | 2.3% |
| Prunus Amygdalus Dulcis Oil | 0.8% |
| Musa Sapientum Fruit Extract | 0.7% |
| Olea Europaea Leaf Extract | 0.5% |
| Sambucus Nigra Flower Extract | 0.5% |
| Camelia Sinensis Leaf Extract | 0.4% |
| Glycyrrhiza Glabra Extract | 0.4% |
| Sodium Benzoate | 0.4% |
| Potassium Sorbate | 1.0% |
| Cyamopsis Tetragonoloba Gum | 1.8% |
| Tocopherol | 2.0% |
| Glycyrrhetinic Acid | 0.4% |
| Phytic Acid | 0.4% |
| Citrus Aurantifolia Peel Oil | 2.0% |
| Helianthus Annuus Seed Oil | 2.3% |
| Rosmarinus Officinalis Leaf Extract | 0.6% |
| Ascorbic Acid | 0.4% |

This moisturising cream may be manufactured by Centisia Laboratorio di Fitocosmesi di Bruno Dott.ssa Laura Francesca & C. S.a.s. of Via XXV Aprile, 83, Laveno-Mombello VA, Italy.

### Example 20: Suncream SPF 15

| **Materie Prime** | **INCI** | **Fase** | **%** | **Peso (Kg)** |
|---|---|---|---|---|
| APIFIL | **PEG-8 BEESWAX** | 1 | 11.000 | 110.00 |
| LABRAFAC | **CAPRYLIC/ CAPRIC TRYGLICERIDE** | 1 | 13.500 | 135.00 |
| OLIVE OIL | **OLEA EUROPAEA FRUIT OIL** | 1 | 5.000 | 50.00 |
| VITAMINA E | **TOCOPHERYL ACETATE** | 3 | 2.000 | 20.00 |
| TOCOPHEROL | **TOCOPHEROL** | 3 | 2.000 | 20.00 |
| PALYTHINE | **SEAWEED EXTRACT** | 1 | 5.000 | 50.00 |
| ACQUA | **AQUA** | 2 | 58.450 | 584.50 |
| HHR | **HYDROXYETHYLCELLULOSE** | 2 | 1.000 | 10.00 |
| EDTA | **EDTA** | 2 | 0.050 | 0.50 |
| ACIDO SORBICO | **SORBIC ACID** | 2 | 0.400 | 4.00 |
| POTASSIO SORBATO | **POTASSIUM SORBATE** | 2 | 1.000 | 10.00 |
| O.E. DI LAVANDA | **LAVANDULA HYBRIDA ABRIALIS OIL** | 3 | 0.100 | 1.00 |
| GUAVA | **PARFUM** | 3 | 0.100 | 1.00 |
| SODIO IDROSSIDO | **SODIUM HYDROXIDE** | 4 | 0.400 | 4.00 |
| | | | | |
| | | | **100.00** | **1000.00** |

This suncream may be manufactured by Oramy S.r.l. of via Nullo, 19, 20129 Milano, Italy.

### Example 21: Suncream SPF 25

| **Materie Prime** | **INCI** | **Fase** | **%** | **Peso (Kg)** |
|---|---|---|---|---|
| APIFIL | **PEG-8 BEESWAX** | 1 | 11.000 | 110.00 |
| LABRAFAC | **CAPRYLIC/ CAPRIC TRYGLICERIDE** | 1 | 9.500 | 95.00 |
| OLIVE OIL | **OLEA EUROPAEA FRUIT OIL** | 1 | 4.000 | 40.00 |
| VITAMINA E | **TOCOPHERYL ACETATE** | 3 | 2.000 | 20.00 |
| TOCOPHEROL | **TOCOPHEROL** | 3 | 2.000 | 20.00 |
| PALYTHINE | **SEAWEED EXTRACT** | 1 | 10.000 | 100.00 |
| ACQUA | **AQUA** | 2 | 58.450 | 584.50 |
| HHR | **HYDROXYETHYLCELLULOSE** | 2 | 1.000 | 10.00 |
| EDTA | **EDTA** | 2 | 0.050 | 0.50 |
| ACIDO SORBICO | **SORBIC ACID** | 2 | 0.400 | 4.00 |
| POTASSIO SORBATO | **POTASSIUM SORBATE** | 2 | 1.000 | 10.00 |
| O.E. DI LAVANDA | **LAVANDULA HYBRIDA ABRIALIS OIL** | 3 | 0.100 | 1.00 |
| GUAVA | **PARFUM** | 3 | 0.100 | 1.00 |
| SODIO IDROSSIDO | **SODIUM HYDROXIDE** | 4 | 0.400 | 4.00 |
| | | | | |
| | | | **100.00** | **1000.00** |

This suncream may be manufactured by Oramy S.r.l. of via Nullo, 19, 20129 Milano, Italy.

### Example 22: Suncream SPF 40

| **Materie Prime** | **INCI** | **Fase** | **%** | **Peso (Kg)** |
|---|---|---|---|---|
| APIFIL | **PEG-8 BEESWAX** | 1 | 8.000 | 80.00 |
| LABRAFAC | **CAPRYLIC/ CAPRIC TRYGLICERIDE** | 1 | 8.500 | 85.00 |
| OLIVE OIL | **OLEA EUROPAEA FRUIT OIL** | 1 | 3.000 | 30.00 |
| VITAMIN E | **TOCOPHERYL ACETATE** | 3 | 2.000 | 20.00 |
| TOCOPHEROL | **TOCOPHEROL** | 3 | 2.000 | 20.00 |
| PALYTHINE | **SEAWEED EXTRACT** | 1 | 16.000 | 160.00 |
| ACQUA | **AQUA** | 2 | 57.450 | 574.50 |
| HHR | **HYDROXYETHYLCELLULOSE** | 2 | 1.000 | 10.00 |
| EDTA | **EDTA** | 2 | 0.050 | 0.50 |
| ACIDO SORBICO | **SORBIC ACID** | 2 | 0.400 | 4.00 |
| POTASSIO SORBATO | **POTASSIUM SORBATE** | 2 | 1.000 | 10.00 |
| O.E. DI LAVANDA | **LAVANDULA HYBRIDA ABRIALIS OIL** | 3 | 0.100 | 1.00 |
| GUAVA | **PARFUM** | 3 | 0.100 | 1.00 |
| SODIO IDROSSIDO | **SODIUM HYDROXIDE** | 4 | 0.400 | 4.00 |
| | | | | |
| | | | **100.00** | **1000.00** |

This suncream may be manufactured by Oramy S.r.l. of via Nullo, 19, 20129 Milano, Italy.

## Claims

1. A composition comprising:
a sunscreening agent consisting essentially of palythine, or salts thereof, in an amount of 0.3% to 25% by weight.

2. A composition according to claim 1 formulated for application to skin.

3. A composition according to claim 1 or claim 2 formulated as a moisturiser.

4. A composition according to any of claims 1 to 3 which comprises
| |
|---|
| emulsifier |
| emollient |
| carrier |
| Active (palythine) 0.3 to 25% by weight |
| water |
| preservative |
and optionally further comprises fragrance.

5. A composition according to any of claims 1 to 4 further comprising antioxidant.

6. A composition according to any of claims 1 to 5 further comprising gelling agent.

7. A composition according to any of claims 1 to 6 which is a sunscreen composition.

8. A composition according to any of claims 1 to 7 for use in the prevention of skin damage, and/or UVA damage, and/or skin aging, and/or UVB damage, and/or reactive oxygen species generation by UVR, wherein said prevention comprises applying said composition to the skin of a subject.

9. A composition for use according to claim 8 wherein said administration is topical.

10. A composition according to any of claims 1 to 7 for use in the prevention of one or more of:
a) UVA damage
b) UVB damage
c) Reactive oxygen species (ROS) damage
d) Formation of cyclobutane pyrimidine dimer (CPD)
e) Formation of 8-oxo-7,8-dihydroguanine (8-oxoGua)
f) Photo-aging

11. A composition according to any of claims 1 to 7 for use as a sunscreen, a UVA protectant, a UVB protectant, or an antioxidant.

## Patentansprüche

1. Zusammensetzung, umfassend:
ein Sonnenschutzmittel, das im Wesentlichen aus Palythin oder Salzen davon in einer Menge von 0,3 % bis 25 Gew.-% besteht.

2. Zusammensetzung nach Anspruch 1, formuliert zum Auftragen auf die Haut.

3. Zusammensetzung nach Anspruch 1 oder Anspruch 2, formuliert als ein Feuchtigkeitsmittel.

4. Zusammensetzung nach einem der Ansprüche 1 bis 3, die umfasst
| |
|---|
| Emulgator |
| Iinderndes Mittel |
| Träger |
| Wirkstoff (Palythin) 0,3 bis 25 Gew.-% |
| Wasser |
| Konservierungsmittel |
und gegebenenfalls weiter Duftstoff umfasst.

5. Zusammensetzung nach einem der Ansprüche 1 bis 4, die weiter ein Antioxidationsmittel umfasst.

6. Zusammensetzung nach einem der Ansprüche 1 bis 5, die weiter ein Geliermittel umfasst.

7. Zusammensetzung nach einem der Ansprüche 1 bis 6, die eine Sonnenschutzzusammensetzung ist.

8. Zusammensetzung nach einem der Ansprüche 1 bis 7 zur Verwendung bei der Prävention von Hautschädigung und/oder UVA-Schädigung und/oder Hautalterung und/oder UVB-Schädigung und/oder der Erzeugung reaktiver Sauerstoffspezies durch UV-Strahlung, wobei die Prävention das Auftragen der Zusammensetzung auf die Haut einer Person umfasst.

9. Zusammensetzung zur Verwendung nach Anspruch 8, wobei die Verabreichung topisch ist.

10. Zusammensetzung nach einem der Ansprüche 1 bis 7 zur Verwendung bei der Prävention eines oder mehrerer von:
a) UVA-Schädigung
b) UVB-Schädigung
c) Schädigung durch reaktive Sauerstoffspezies (ROS)
d) Bildung von Cyclobutan-pyrimidin-Dimer (CPD)
e) Bildung von 8-Oxo-7,8-dihydroguanin (8-OxoGua)
f) Foto-Alterung

11. Zusammensetzung nach einem der Ansprüche 1 bis 7 zur Verwendung als ein Sonnenschutz-, ein UVA-Schutzmittel, ein UVB-Schutzmittel oder ein Antioxidationsmittel.

## Revendications

1. Composition comprenant :
un agent d'écran solaire constitué essentiellement de palythine, ou de sels de cette dernière, en une quantité de 0,3 % à 25 % en poids.

2. Composition selon la revendication 1 formulée pour application sur la peau.

3. Composition selon la revendication 1 ou revendication 2 formulée en tant que crème hydratante.

4. Composition selon l'une quelconque des revendications 1 à 3 qui comprend
| |
|---|
| émulsifiant |
| émollient |
| porteur |
| Actif (palythine) 0,3 à 25 % en poids |
| eau |
| conservateur |
et comprend éventuellement en outre un parfum.

5. Composition selon l'une quelconque des revendications 1 à 4 comprenant en outre un antioxydant.

6. Composition selon l'une quelconque des revendications 1 à 5 comprenant en outre un agent gélifiant.

7. Composition selon l'une quelconque des revendications 1 à 6 qui est une composition d'écran solaire.

8. Composition selon l'une quelconque des revendications 1 à 7 pour utilisation dans la prévention des lésions cutanées, et/ou les dommages causés par les UVA, et/ou le vieillissement de la peau, et/ou les dommages causés par les UVB, et/ou la génération d'espèces réactives de l'oxygène par les UVR, dans laquelle ladite prévention comprend l'application de ladite composition sur la peau d'un sujet.

9. Composition pour utilisation selon la revendication 8 dans laquelle ladite administration est topique.

10. Composition selon l'une quelconque des revendications 1 à 7 pour utilisation dans la prévention d'une ou plusieurs parmi :
a) les dommages causés par les UVA
b) les dommages causés par les UVB
c) les dommages causés par la génération d'espèces réactives de l'oxygène (ROS)
d) la formation de dimère de cyclobutane-pyrimidine (CPD)
e) la formation de 8-oxo-7,8-dihydroguanine (8-oxoGua)
f) le photo-vieillissement

11. Composition selon l'une quelconque des revendications 1 à 7 pour utilisation en tant qu'écran solaire, protecteur contre les UVA, protecteur contre les UVB ou antioxydant.
